# EUROPEAN PATENT SPECIFICATION

(11) **EP 4 034 555 B1**
(45) Date of publication and mention of the grant of the patent: **12.02.2025**
(21) Application number: 20788997.3
(22) Date of filing: 23.09.2020
(51) Int. Cl.: C07K 14/725, A61P 35/00, A61K 35/17, A61K 39/00

(54) **DAP10/DAP12 FUSION POLYPEPTIDES**
DAP10/DAP12-FUSIONSPOLYPEPTIDE
POLYPEPTIDES DE FUSION DAP10/DAP12

(30) Priority: 23.09.2019 GB 201913697
(43) Date of publication of application: 03.08.2022
(73) Proprietor: King's College London, London WC2R 2LS (GB)
(72) Inventor: MAHER, John, London WC2R 2LS (GB); DAVIES, David Marc, London WC2R 2LS (GB)
(74) Representative: Greaves Brewster LLP
(86) International application number: PCT/EP2020/076566
(87) International publication number: WO 2021/058563

(56) References cited:
- WO-A1-2019/077165
- WO-A1-2019/182425
- WO-A2-2020/168298
- US-A1- 2018 002 438
- TSUKASA NABEKURA ET AL: "Cutting Edge: NKG2D Signaling Enhances NK Cell Responses but Alone Is Insufficient To Drive Expansion during Mouse Cytomegalovirus Infection", THE JOURNAL OF IMMUNOLOGY, vol. 199, no. 5, 31 July 2017 (2017-07-31), US, pages 1567 - 1571, XP055741836, ISSN: 0022-1767, DOI: 10.4049/jimmunol.1700799
- BRIAN RABINOVICH ET AL: "NKG2D splice variants: a reexamination of adaptor molecule associations", IMMUNOGENETICS, SPRINGER, BERLIN, DE, vol. 58, no. 2-3, 1 April 2006 (2006-04-01), pages 81 - 88, XP019331711, ISSN: 1432-1211, DOI: 10.1007/S00251-005-0078-X

## Description

### TECHNICAL FIELD

This invention relates to fusion polypeptides comprising a DNAX-activating protein 10 (DAP10) polypeptide and a DNAX-activating protein 12 (DAP12) polypeptide. The disclosure also relates to cells comprising such fusion polypeptides and their use in treating cancer.

### BACKGROUND

Immunotherapy using chimeric antigen receptor (CAR)-engineered T-cells has proven transformative in the management of B-cell malignancy and multiple myeloma. However, application of this technology to solid tumour immunotherapy is impeded by the lack of tumour-selective targets. Most tumour antigens are intracellular and thus cannot easily be recognised by CAR T-cells. Consequently, most solid tumour directed CARs that are currently under development engage targets that are upregulated in tumour cells, but which are found at lower levels in normal tissues.

One of the few target groups that exhibits a high degree of tumour selectivity are the NKG2D ligands. In man, these comprise a group of 8 stress-induced proteins (MICA, MICB, ULBP1-6) that are aberrantly expressed on virtually all tumour cell types. Moreover, NKG2D ligands are also found on tumour associated stromal elements such as endothelium, regulatory T-cells and myeloid derived suppressor cells (Parihar, R., et al., 2019, Cancer Immunol. Res. 7(3):363-375; Schmiedel & Mandelboim, 2018, Front. Immunol. (9)2040). Mice that are genetically deficient in NKG2D demonstrate impaired immunosurveillance for both epithelial and lymphoid malignancies. Evidence that NKG2D ligands are safe therapeutic targets is supported by the fact that they are not found in healthy tissues. Moreover, ongoing clinical trials involving NKG2D-targeted CARs have not revealed significant safety issues, even when combined with fludarabine/ cyclophosphamide lymphodepleting chemotherapy (see https://www.celyad.com/en/news/celyad-presents-update-on-autologous-allogeneic-nkg2d-based-car-t-therapies-in-solid-tumors).

The NKG2D receptor is naturally expressed by natural killer (NK) and some T-cell populations. Each NKG2D homodimer associates with two homodimeric DAP10 adaptor molecules via complementary charged amino acids within the plasma membrane. This interaction is required for cell surface expression and function of NKG2D. DAP10 resembles CD28 in its ability to provide co-stimulation via phosphatidylinositol 3-kinase but, importantly, it lacks a p56ick binding motif that promotes the unwanted recruitment of regulatory T-cells (Kofler, et al., 2011, Mol. Ther. 19:760-767). Potency of DAP10 co-stimulation is underscored by its continued ability to signal following internalisation. However, since DAP10 lacks an immunoreceptor tyrosine-based activation motif (ITAM), NKG2D engagement does not lead to full T-cell activation.

A variety of CARs have been developed which use different methods to provide ITAM-dependent signal 1 in addition to co-stimulation (also known as signal 2), as both signal types are necessary to elicit full T-cell activation. The first NKG2D targeted CAR was developed by Sentman et al. and consists of a fusion of NKG2D to CD3ζ (Zhang et al, 2005, Blood 106:1544-1551). Although nominally a first-generation CAR, it associates with endogenous DAP10 in T-cells, meaning that both signals 1 and 2 are provided. This CAR is currently undergoing clinical development by Celyad S.A. as Cyad-01. More recently, Chang et al. (2013, Cancer Res. 73:1777-1786) engineered NK cells to co-express an identical CAR in addition to exogenous DAP10. Two further NKG2D CARs have also been described that incorporate either 4-1BB (Song et al., 2013, Hum. Gene Ther. 24:295-305) or CD28 (Lehner et al., 2012, PLoS One 7:e31210) to provide alternative forms of co-stimulation instead of that provided by DAP10. All of these CARs have enabled T-cell mediated tumour cell killing accompanied by cytokine production while the CAR described by Chang et al. also demonstrated transient in vivo anti-tumour activity. US 2018/002438 A1 discloses CARs comprising either DAP10 or DAP12, including fusions with NKG2D.

### SUMMARY OF THE INVENTION

One isoform of mouse NKG2D found in NK cells can associate in trans with both DAP10 and DAP12 (which contains a single ITAM), thereby potentially delivering both signal 1 and signal 2 for T-cell activation. Based on this observation, we have engineered a fusion polypeptide comprising both DAP10 and DAP12. When co-expressed with NKG2D, the fusion polypeptide and NKG2D co-associate in the plasma membrane via complementary charged amino acid residues to form a compact adaptor-based CAR which drives full T-cell activation.

Thus, the present invention provides a fusion polypeptide comprising:
(i) a DNAX-activating protein 10 (DAP10) polypeptide comprising the sequence of SEQ ID NO: 1, or a functional variant thereof, wherein the functional variant
   (a) comprises an amino acid sequence having at least 80% sequence identity to SEQ ID NO: 1, or
   (b) is a truncated version of SEQ ID NO: 1 comprising or consisting of amino acids 19-93, 19-69, 1-71, 19-71, 19-48, 49-69, 49-93, or 70-93 of SEQ ID NO: 1; and
(ii) a DNAX-activating protein 12 (DAP12) polypeptide comprising the sequence of SEQ ID NO: 9, or a functional variant thereof, wherein the functional variant
   (a) comprises an amino acid sequence having at least 80% sequence identity to SEQ ID NO: 9, or
   (b) is a truncated version of SEQ ID NO: 9 comprising or consisting of amino acids 22-113, 62-113, 22-61 or 41-61 of SEQ ID NO: 9.

The DAP10 polypeptide (or functional variant thereof) and DAP12 polypeptide (or functional variant thereof) may be directly fused together, or may be joined by a linker. The fusion polypeptide may further comprise N-terminal or C-terminal amino acid sequences, for example, to assist with detection or purification, to improve expression or to increase half-life.

In one embodiment, the fusion polypeptide of the invention has the formula, from N-terminus to C-terminus:

A-B-C-D-E,

wherein
A = an optional N-terminal sequence
B = a DAP10 polypeptide or functional variant thereof
C = an optional linker sequence
D = a DAP12 polypeptide or functional variant thereof
E = an optional C-terminal sequence.

In one embodiment, the fusion polypeptide does not comprise or consist of SEQ ID NO: 1 of WO 2019/182425 (referred to as SEQ ID NO: 84 herein). In some embodiments, the fusion polypeptide does not comprise an anti-EpCAM peptide. In some embodiments, the fusion polypeptide does not comprise an EpCAM specific antigen receptor. In some embodiments, the fusion polypeptide does not comprise or consist of SEQ ID NO: 9 of WO 2019/182425 (referred to as SEQ ID NO: 85 herein). In some embodiments, the DAP10 polypeptide or functional variant thereof does not comprise or consist of SEQ ID NO: 85. In some embodiments, the fusion polypeptide does not comprise or consist of SEQ ID NO: 11 of WO 2019/182425 (referred to as SEQ ID NO: 86 herein). In some embodiments, the DAP12 polypeptide or functional variant thereof does not comprise or consist of SEQ ID NO: 86. In some embodiments, the fusion polypeptide does not comprise or consist of both SEQ ID NO: 85 and SEQ ID NO: 86.

In other aspects, the invention relates to a nucleic acid molecule (e.g., an isolated nucleic acid molecule), including DNA and RNA molecules, that encodes a fusion polypeptide as described herein. Such nucleic acid molecules may optionally also encode a NKG2D polypeptide or functional variant thereof.

Also provided by the invention are vectors, particularly expression vectors, comprising a nucleic acid molecule of the invention.

The invention also provides host cells comprising a nucleic acid and/or vector that encodes a fusion polypeptide as described herein. Host cells comprising the fusion polypeptide of the invention are also provided herein.

The invention also provides a method for making an immunoresponsive cell, comprising the steps of:
(i) transducing the nucleic acid or vector of the invention into the immunoresponsive cell; and
(ii) culturing the immunoresponsive cell such that a fusion polypeptide is expressed and associates with a NKG2D polypeptide to form a CAR.

Also provided is a pharmaceutical composition comprising the fusion polypeptide, nucleic acid molecule, vector, host cell, or immunoresponsive cell of the invention, and further comprising a pharmaceutically or physiologically acceptable diluent and/or carrier.

The invention also provides a fusion polypeptide, nucleic acid molecule, vector, host cell, immunoresponsive cell or pharmaceutical composition according to the invention for use in the treatment of a pathological disorder, optionally wherein the pathological disorder is cancer.

### BRIEF DESCRIPTION OF THE FIGURES

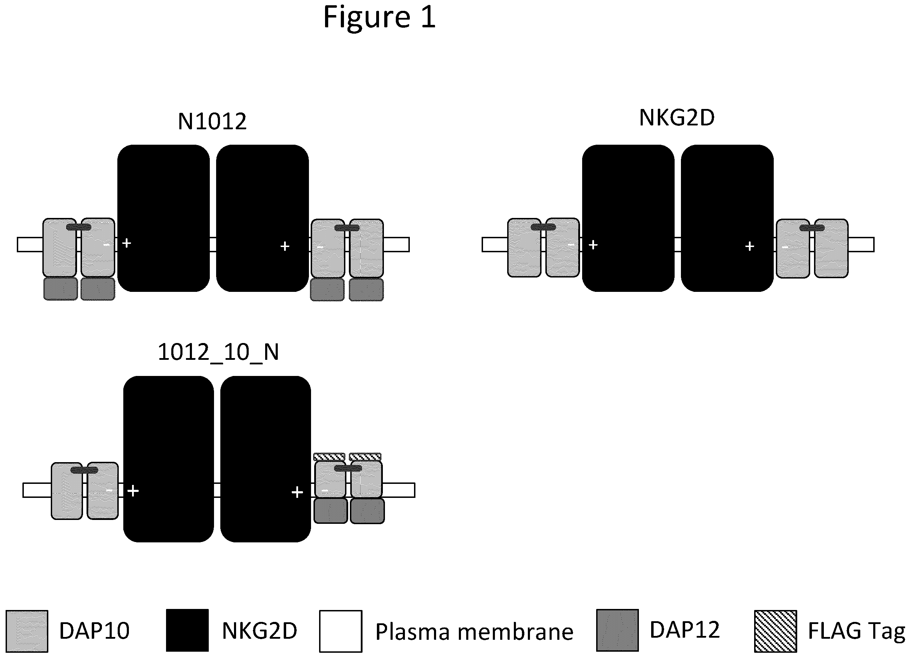

Figure 1 is a schematic demonstrating the structure of each of the constructs that have been generated. N1012 comprises a complex comprising an exogenous human NKG2D protein and fused exogenous DAP10/12 homodimers according to the invention. N1012 comprises SEQ ID NO: 64, which is described in Table 2. The NKG2D complex comprises an exogenous human NKG2D protein which interacts with endogenous DAP10 already present in the cell, and is provided for comparative purposes only.

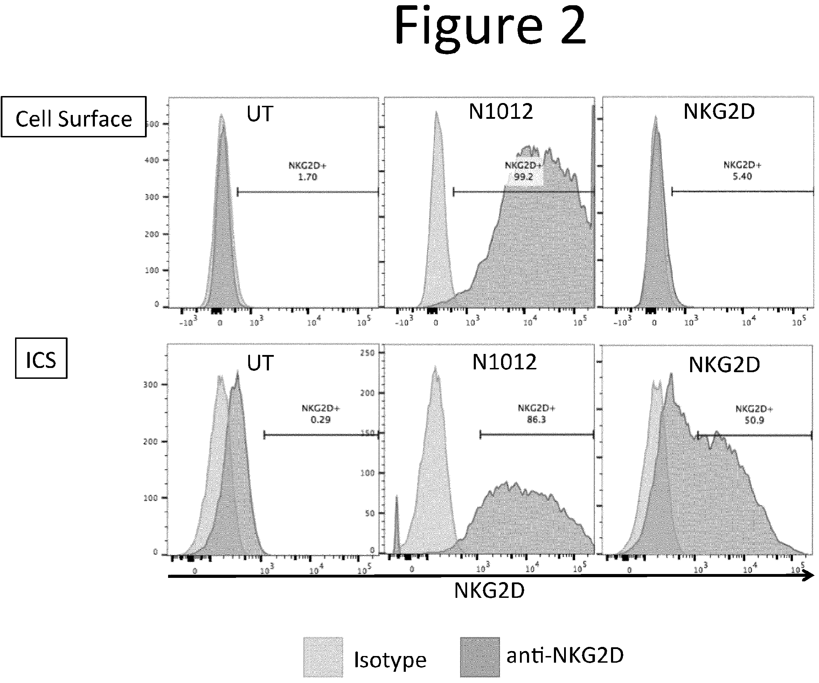

Figure 2 shows the expression of NKG2D at both the cell surface and intracellularly (ICS) in 293T cells three days after transfection with either the retroviral plasmid expressing N1012 (shown schematically in Figure 1) or NKG2D alone, making comparison with untransduced (UT) 293T cells.

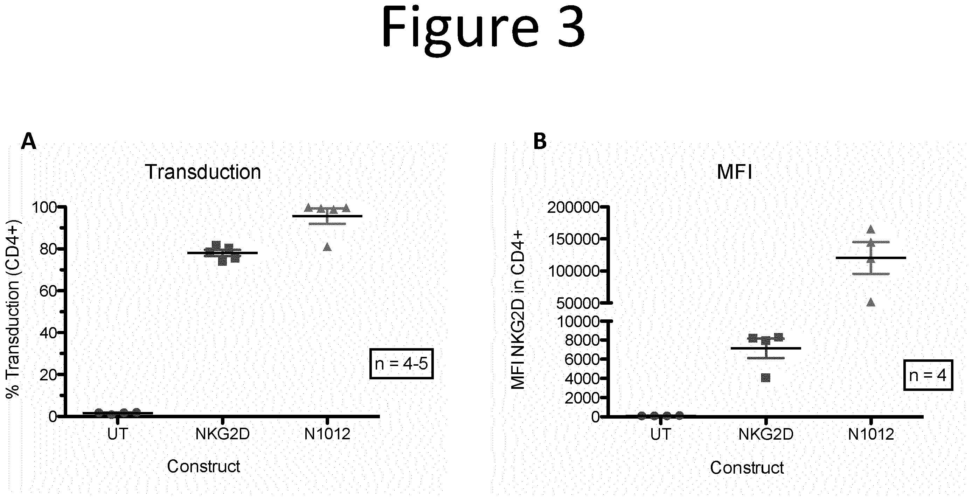

Figure 3 shows the transduction percentage (Figure 3A) and median fluorescence intensity (MFI - Figure 3B) of cell surface NKG2D expression in the CD4⁺ subset of T-cells, following transduction of activated unfractionated human T-cells with retroviral vectors that encode for NKG2D alone or N1012. The expression and MFI in UT T-cells is also shown for comparison.

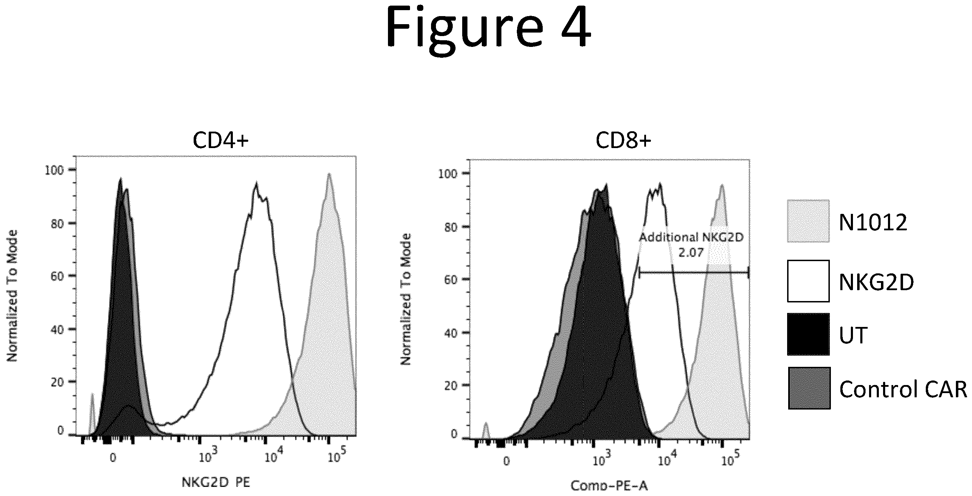

Figure 4 shows a representative expression pattern of cell surface NKG2D within the CD4⁺ and CD8⁺ subset of activated unfractionated T-cells transduced to express N1012, NKG2D or a control CAR. NKG2D expression within UT T-cells is provided as a comparison.

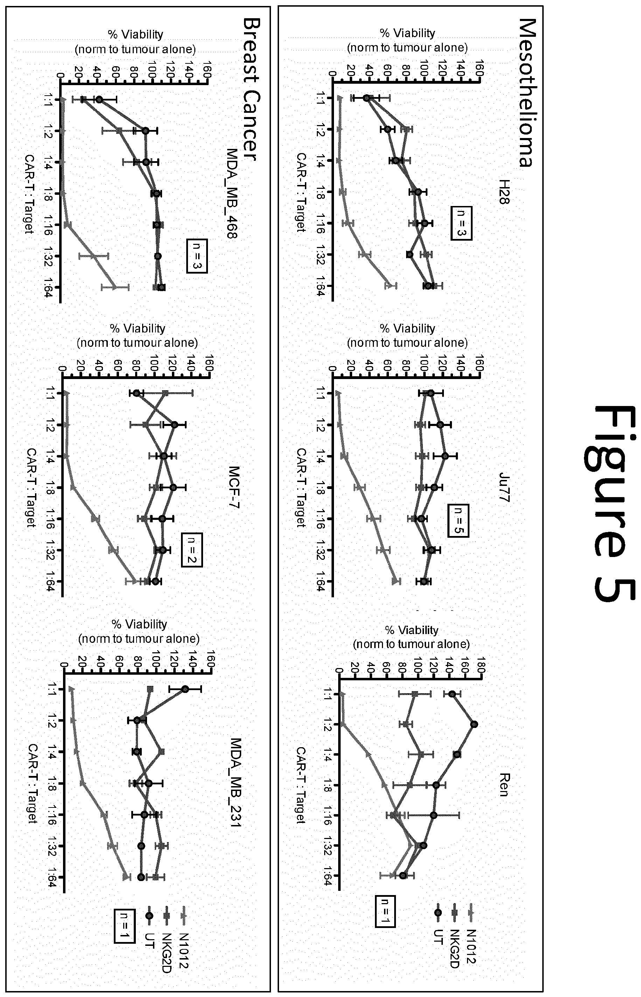

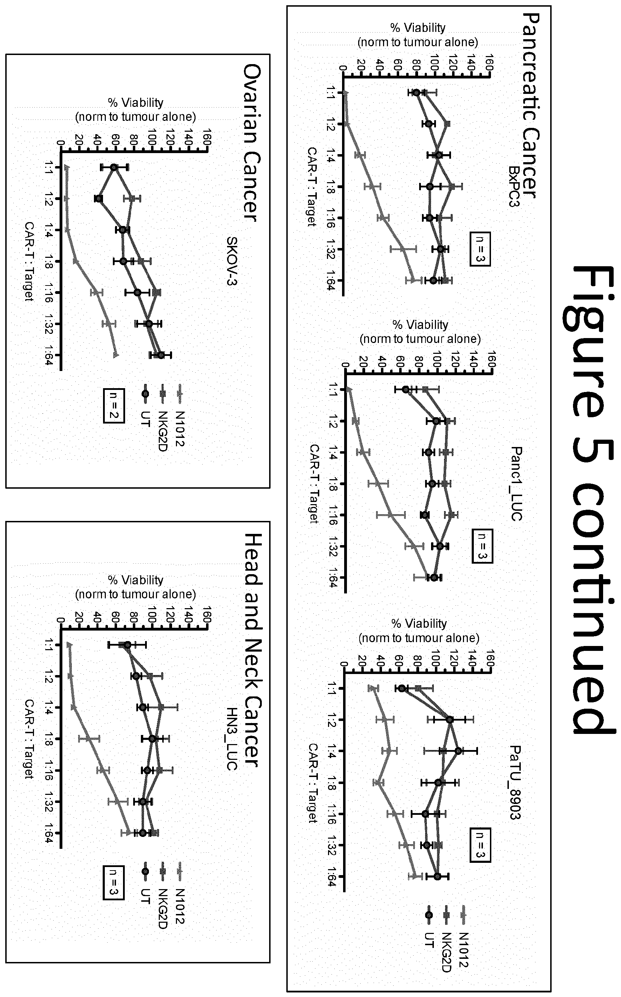

Figure 5 shows the viability of 11 different tumour cell lines after co-culture with N1012⁺, NKG2D⁺ or UT T-cells at varying CAR T-cell:target ratios. Tumour cell viability was assessed after 72 hours using an MTT assay and is expressed as a percentage of that observed in the absence of T-cell co-culture.

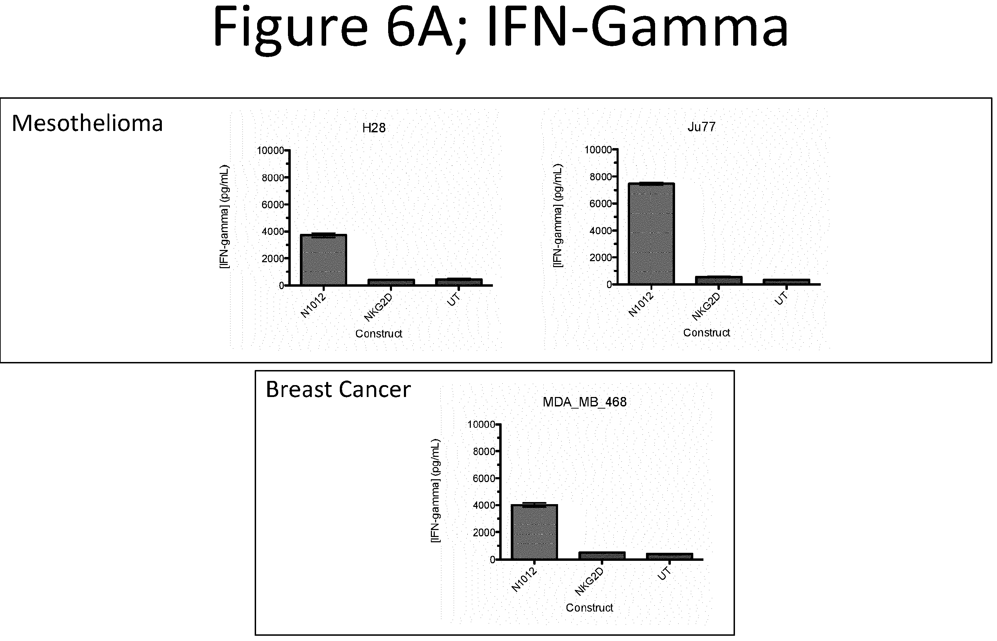

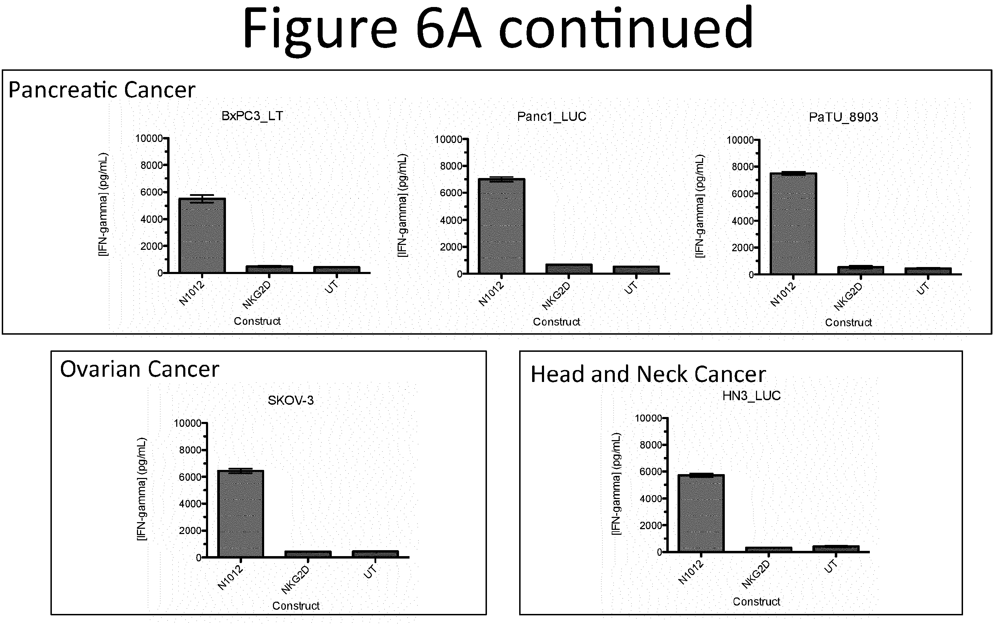

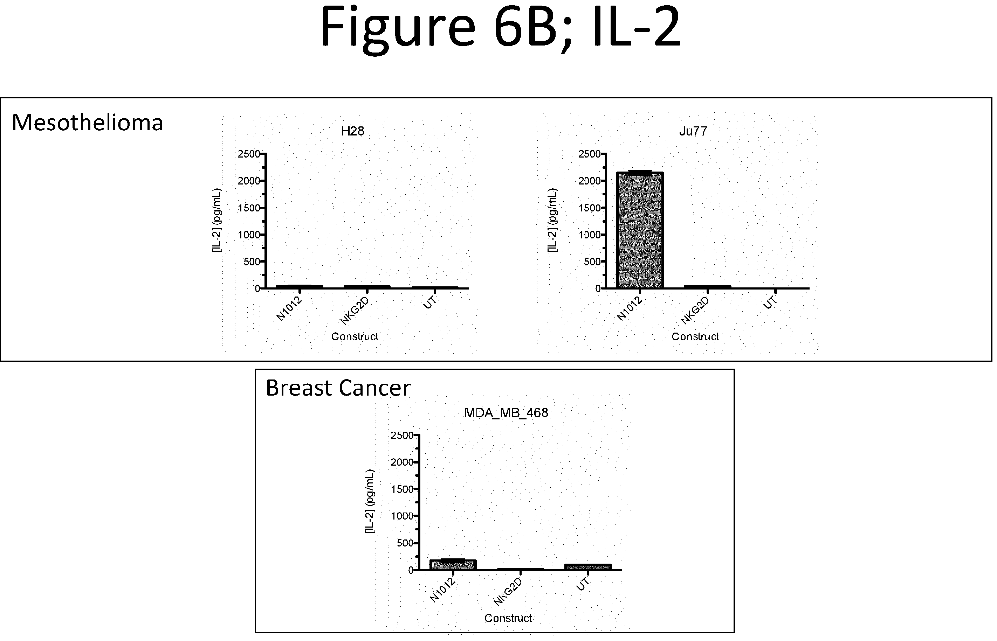

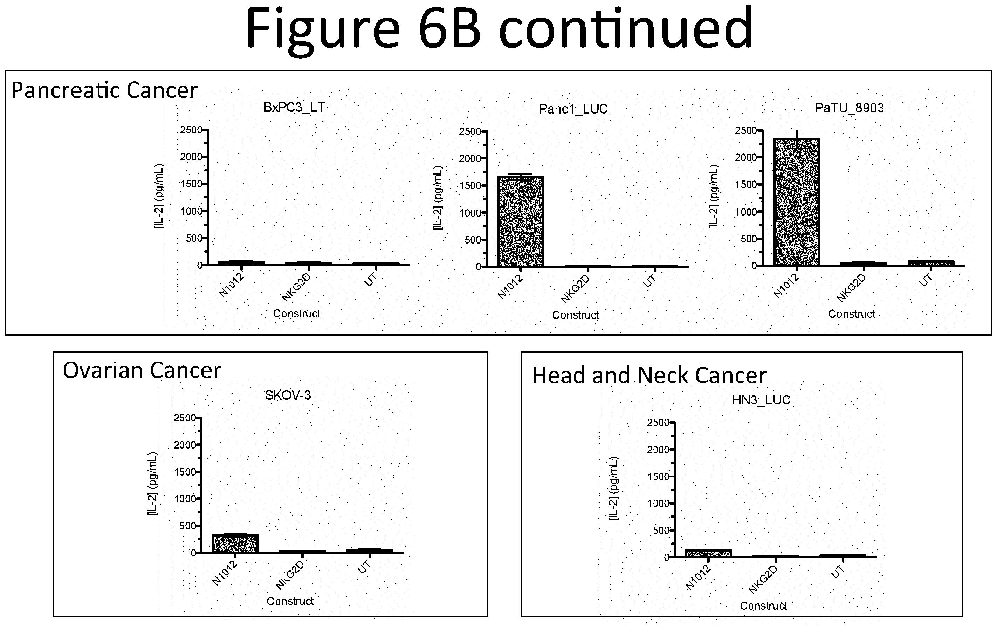

Figure 6 shows the levels of cytokine (IFN-y (Figure 6A) and IL-2 (Figure 6B)) secreted by N1012, NKG2D and UT T-cells during co-culture with 8 different tumour cell lines at a 1:1 CAR T-cell:target ratio. Co-culture supernatants were removed after 72 hours and assessed for cytokine presence by enzyme-linked immunosorbent assay (ELISA).

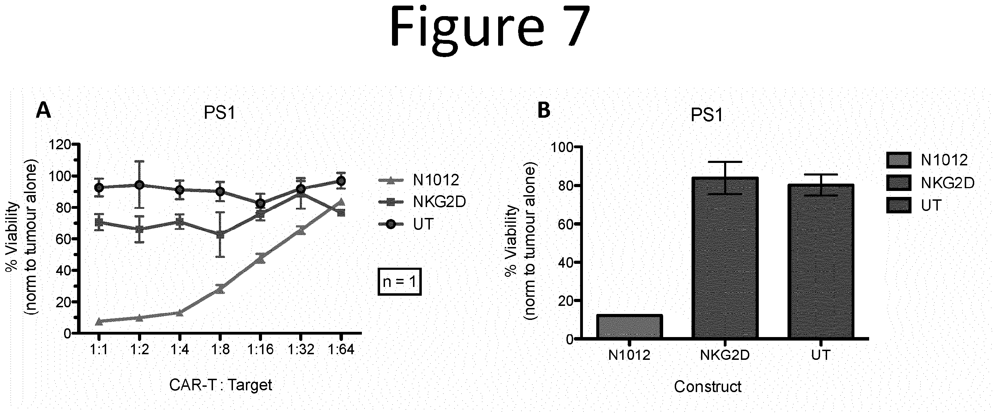

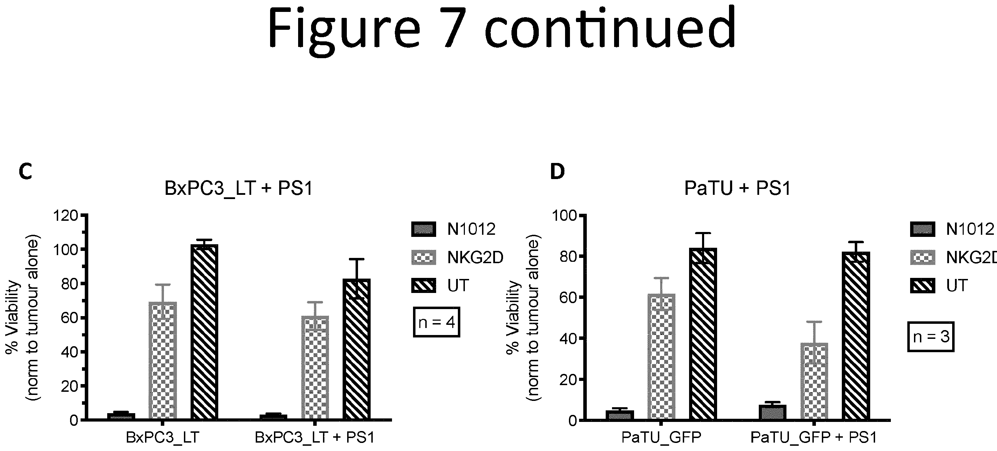

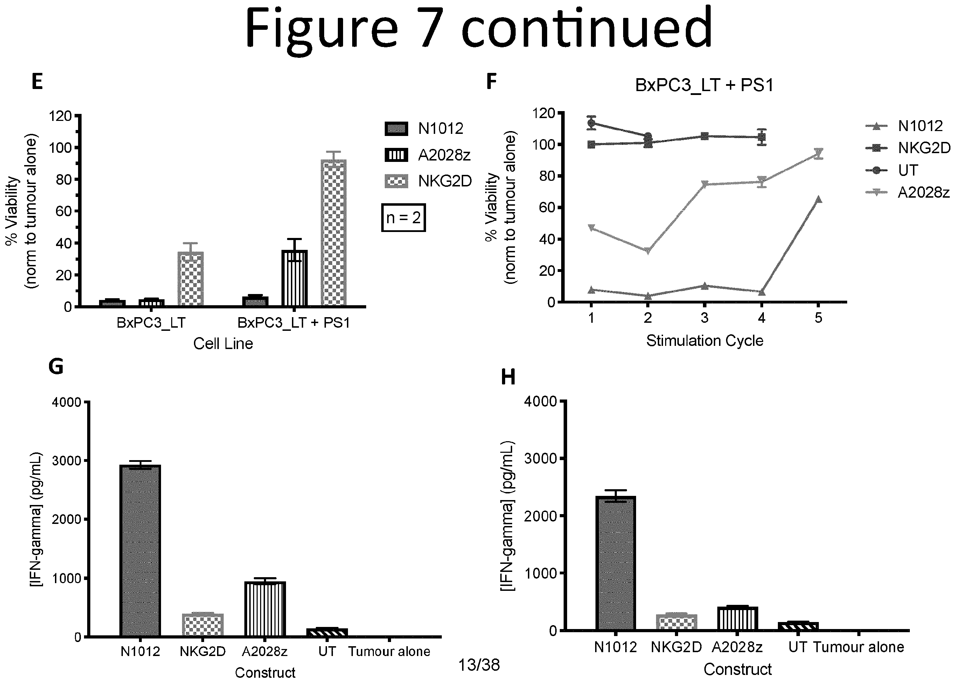

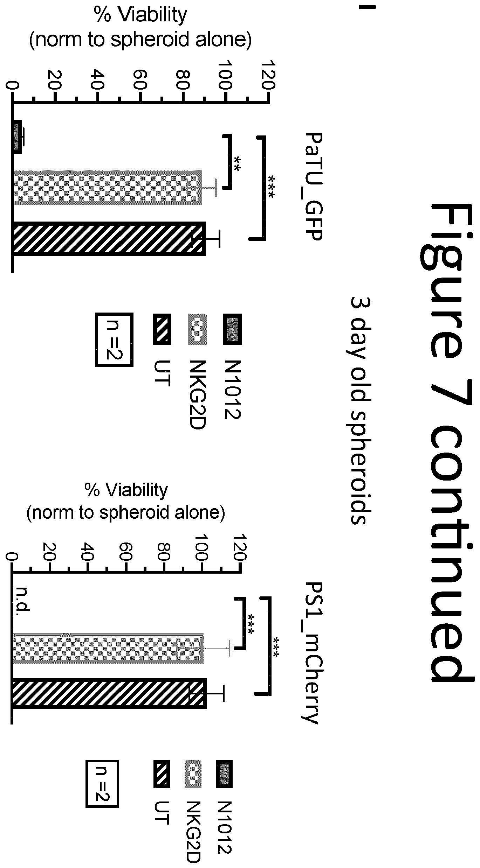

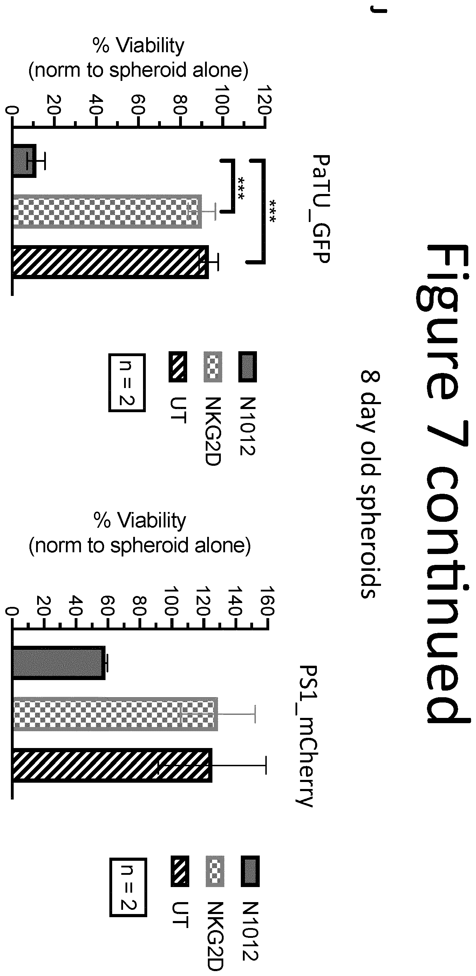

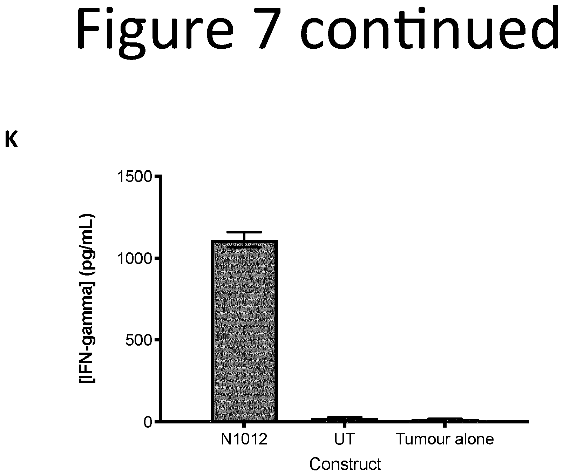

Figure 7 shows the viability of tumour and pancreatic stellate PS1 after co-culture with N1012⁺, NKG2D⁺ or UT T-cells at varying CAR T-cell:target ratios. Figure 7A shows the viability of pancreatic stellate PS1 cells after co-culture with N1012⁺, NKG2D⁺ or UT T-cells at varying CAR T-cell:target ratios. When added at a 1:1 CAR T-cell:target ratio, N1012⁺ T-cells also demonstrate potent lysis of monolayers consisting of both PS1 and BxPC3 cells grown together at a 1:1 ratio (Figure 7B). This contrasts with the lack of efficacy observed upon the addition of either NKG2D⁺ or UT T-cells. In both cases, target cell viability was assessed after 48 hours by MTT assay and is expressed as a percentage of that observed in the absence of T-cell co-culture. The viability of tumour cell and stromal cell monolayers after co-culture with N1012⁺, NKG2D⁺ or UT T-cells at a 1:1 CAR T-cell:target ratio is shown in Figure 7C (BxPC3_LT + PS1) and Figure 7D (PaTU + PS1). Whereas potent lysis of both tumour and stromal cells was observed with N1012⁺ T-cells, a minimal reduction in target cell viability was observed when co-cultured with either NKG2D or UT T-cells (Figure 7C-D). The viability of tumour cell and stromal cell monolayers after co-culture with N1012⁺, A2028z⁺, NKG2D⁺ or UT T-cells at a 1:1 CAR T-cell:target ratio is shown in Figures 7E and Figure 7F (after up to 5 re-stimulations). Figures 7G and H shows the level of IFN-y secreted by N1012⁺, A2028z⁺, NKG2D⁺ and UT T-cells during the co-cultures described above. The level of IFN-y secreted from the tumour alone was determined as a negative control. Figures 7I and J show the viability of tumour spheroids following co-culture with N1012⁺, NKG2D⁺ or UT T-cells. Figure 7K shows the levels of IFN-y secreted by N1012⁺, UT T-cells and tumour alone during the co-cultures with tumour spheroids.

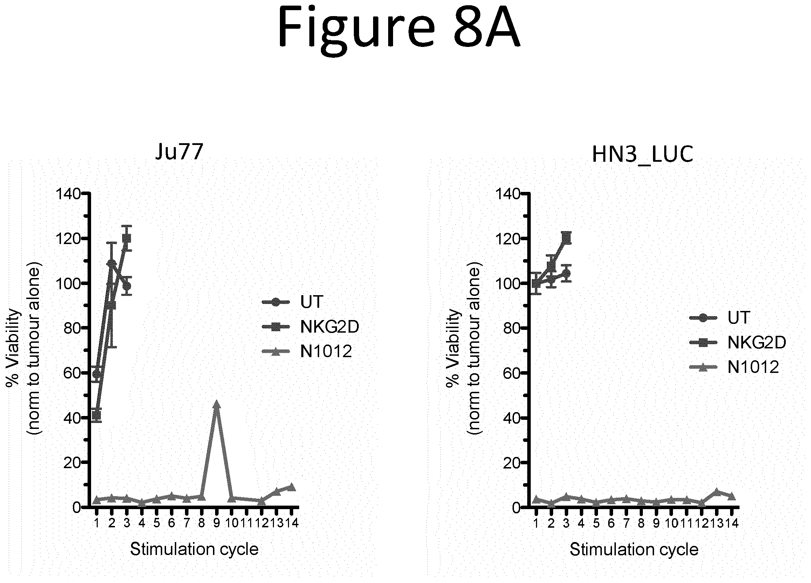

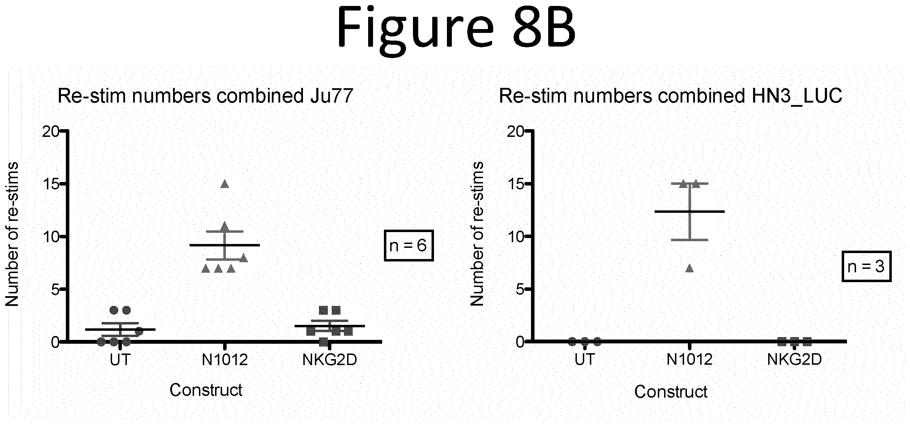

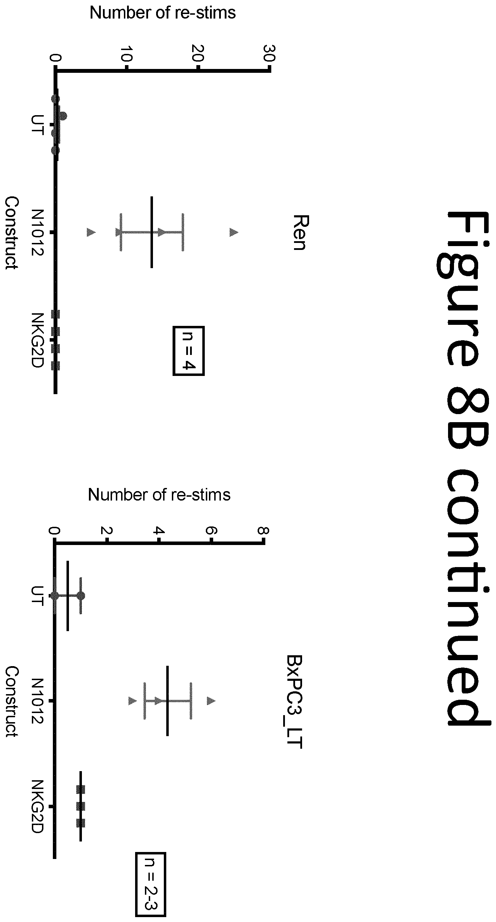

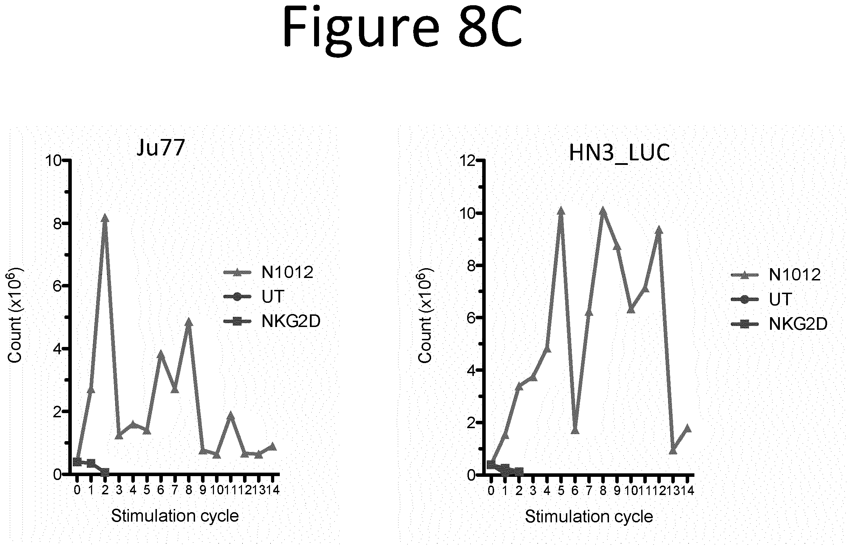

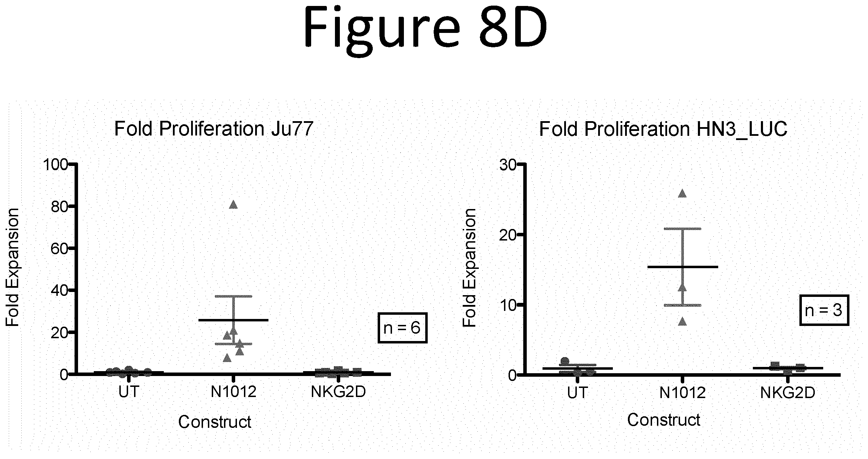

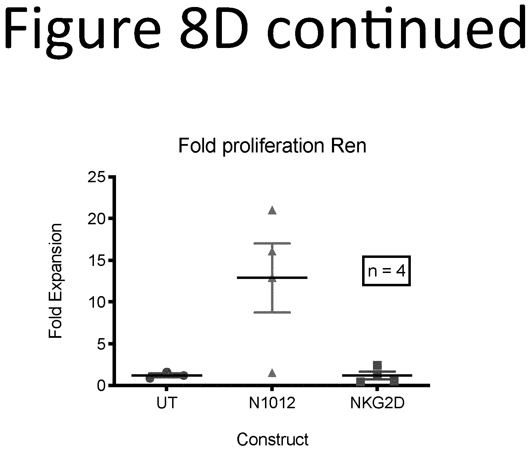

Figure 8 shows the ability of T-cells expressing N1012 to undergo multiple rounds of repeated stimulation ('re-stimulation'), when compared to UT T-cells, or those expressing NKG2D alone. T-cells expressing N1012 are able to mediate lysis of both mesothelioma (Ju77) cells and head and neck (HN3_LUC) cells (Figure 8A) through multiple cycles of stimulation (Figure 8B), during which they demonstrate substantial proliferation (Figure 8C) and fold expansion of T-cells (Figure 8D). Figures 8B and 8D also show that T-cells expressing N1012 can undergo repeated rounds of stimulation and demonstrate substantial proliferation when cultured with Ren or BxP3_LT-cells. In contrast, UT T-cells or those expressing NKG2D alone demonstrate minimal target cell lysis or proliferation.

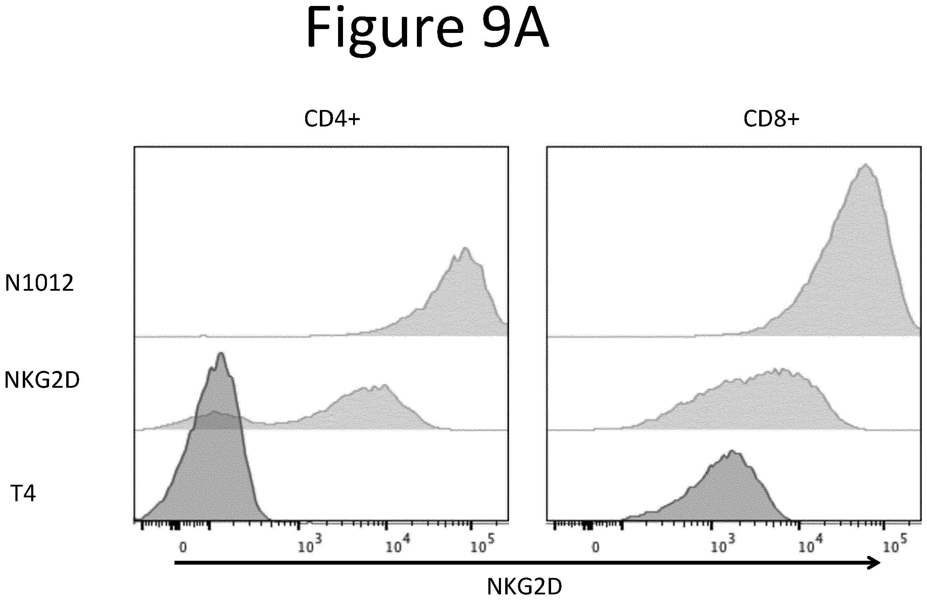

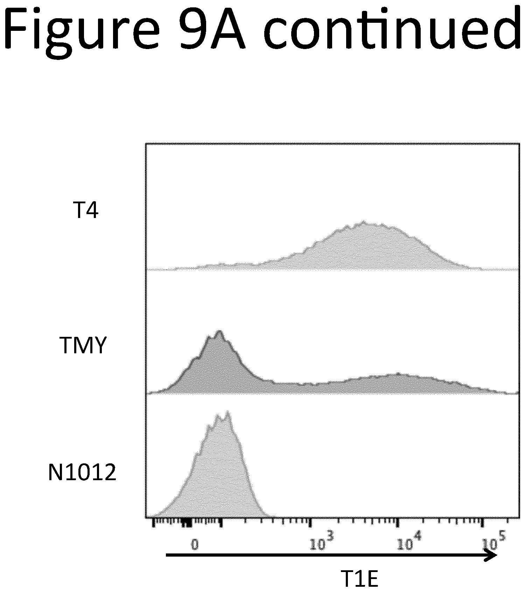

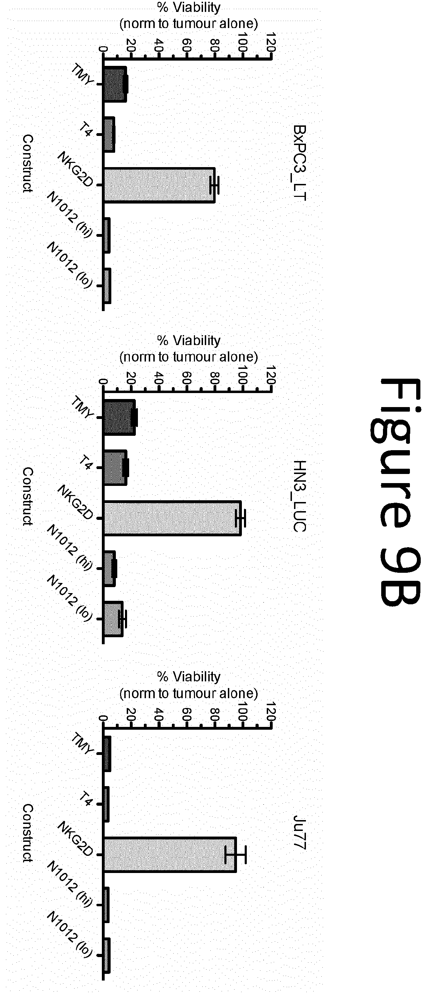

Figure 9 shows the engineering by retroviral transduction of CAR T-cells for *in vivo* evaluation in tumour-bearing mice. Figure 9A shows cell surface expression of NKG2D in CD4⁺ and CD8⁺ T-cells following transduction with either N1012 or NKG2D. Comparison was made with T-cells from the same donor expressing the pan-ErbB targeting CAR, T4, as described by Davies et al., 2012, Mol. Med. 18:565-576). A second pan-ErbB targeting CAR, designated TMY, that lacks the 4αβ domain contained within T4 and also has a slightly altered CD28 hinge (in which the MYPPPY sequence is replaced with the 10 amino acid linear myc tag sequence) was also used. Figure 9B shows the *in vitro* cytotoxic function of residual T-cells against the indicated tumour cell lines following adoptive transfer to mice.

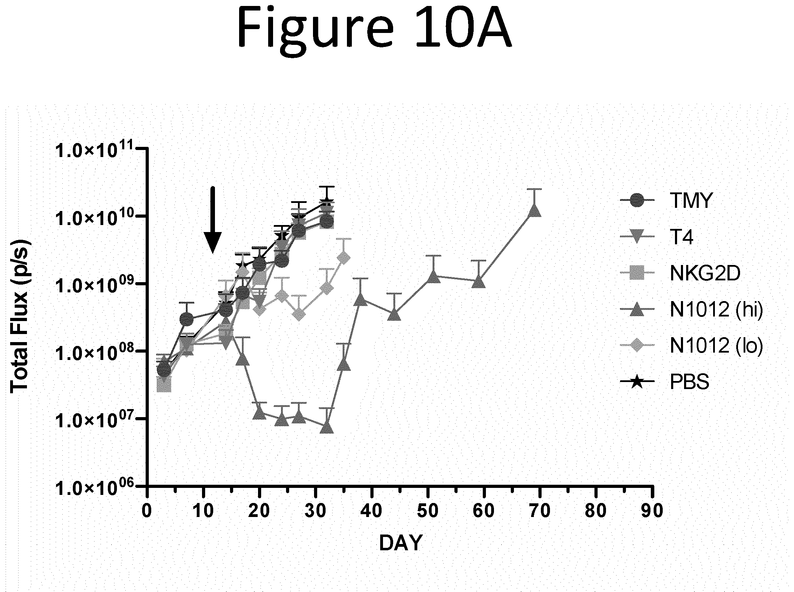

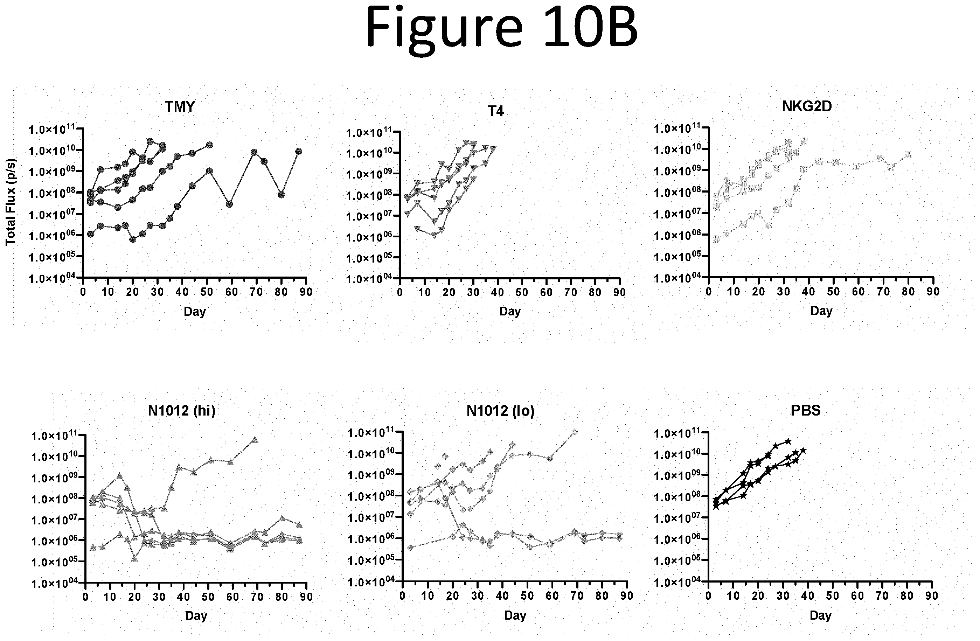

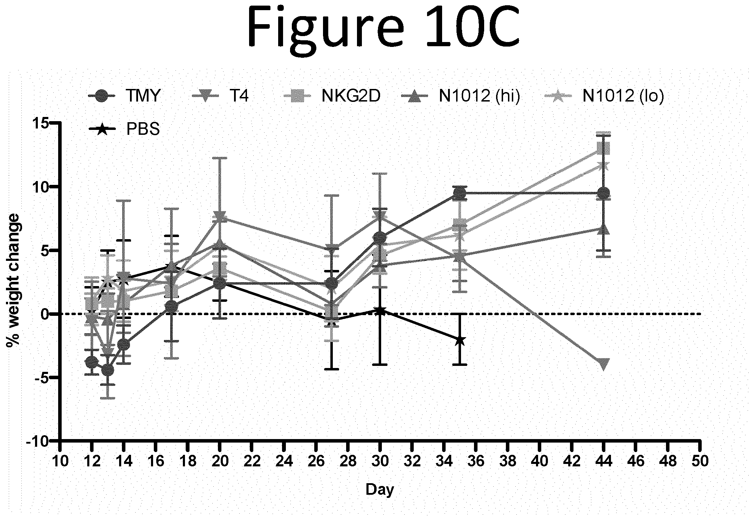

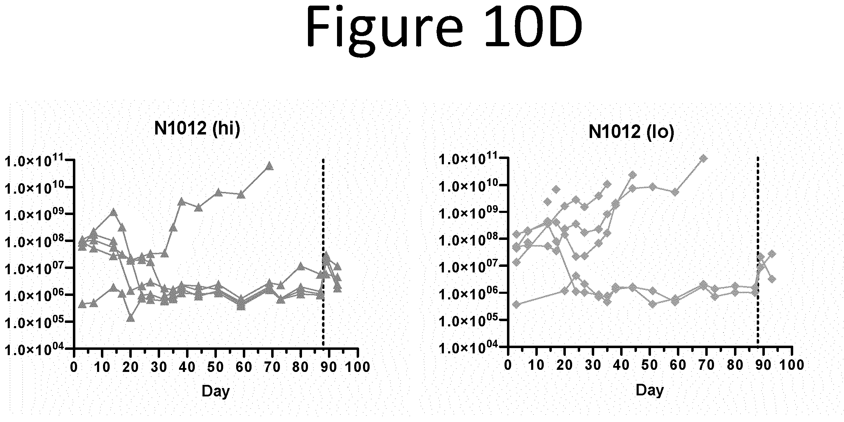

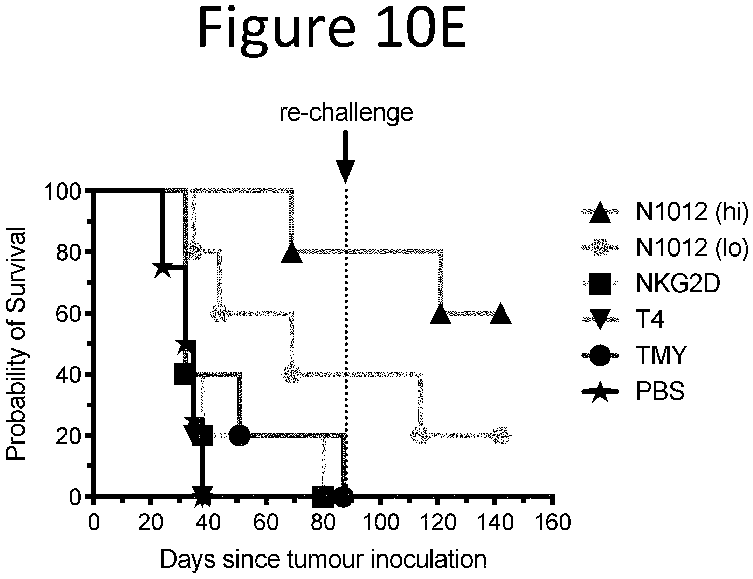

Figure 10 shows the growth of ffLUC-tagged BxPC3 cells *in vivo* in NSG mice, as ascertained by bioluminescence imaging (BLI). Pooled BLI emission from each group of mice is shown in Figure 10A. Serial bioluminescence emission from individual mice is shown in Figure 10B. Weight of mice is shown in Figure 10C. Serial bioluminescence emission from individual mice following tumour re-challenge on day 88 is shown in Figure 10D. A survival curve of the experiment, which was ended after 145 days, is shown in Figure 10E.

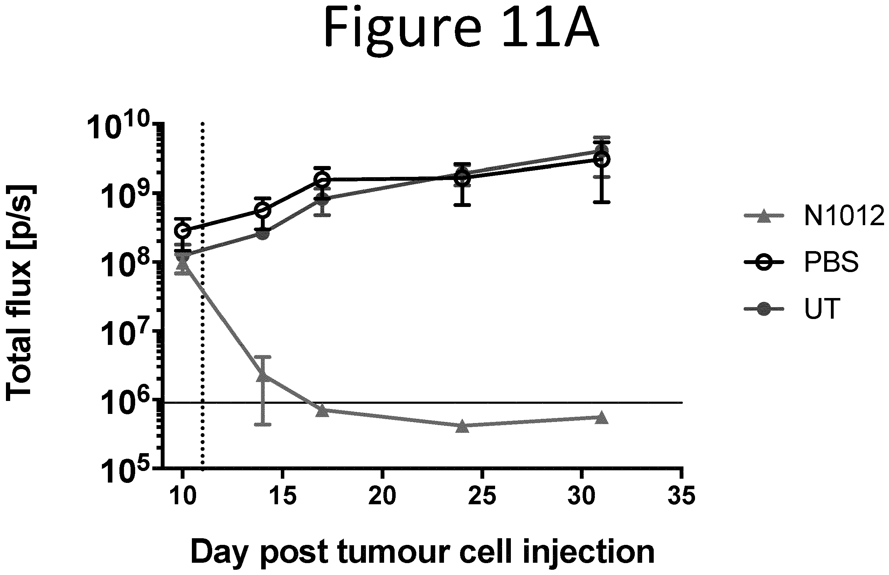

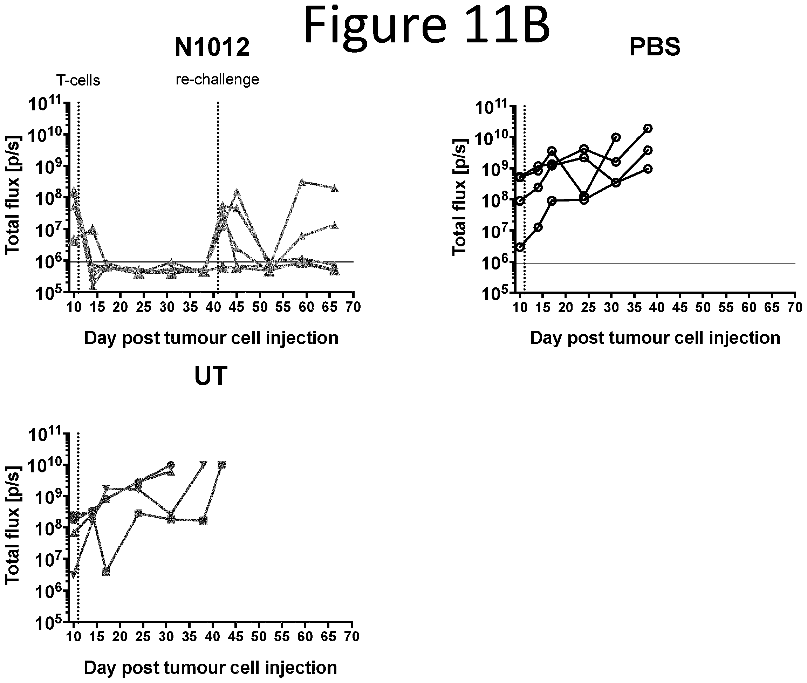

Figure 11 shows the results of another experiment to show the growth of ffLUC-tagged BxPC3 cells *in vivo* in NSG mice, as ascertained by bioluminescence imaging (BLI). BLI average total flux (photons/second) per treatment group is shown in Figure 11A, and BLI total flux (photons/second) per individual mouse is shown in Figure 11B. Mice that were tumour free at day 41 (29 days after T-cell infusion) were re-challenged i.p. (shown as a dotted line) with ffLUC-tagged BxPC3 cells.

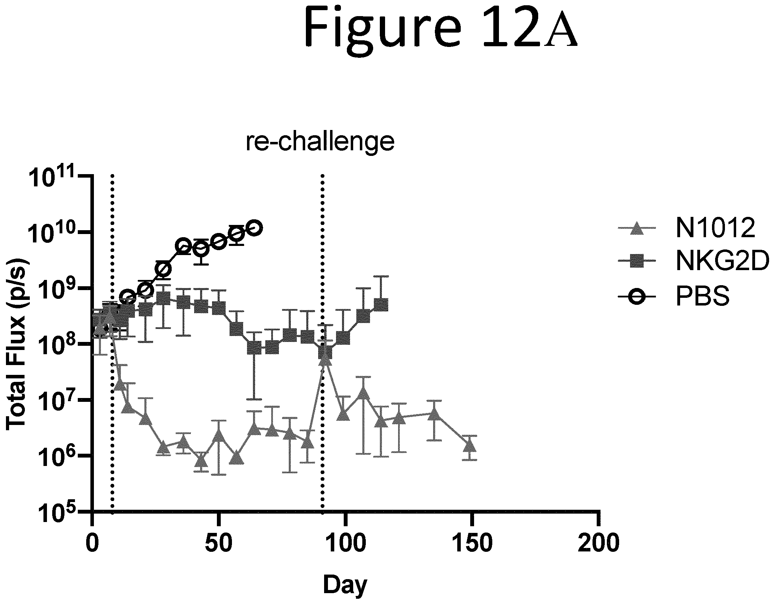

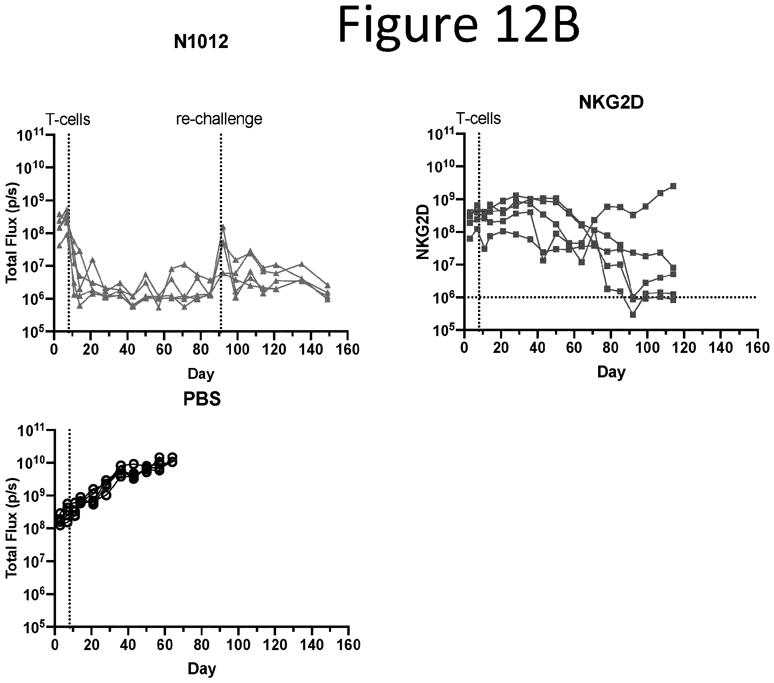

Figure 12 shows the growth of ffLUC-tagged H226 malignant mesothelioma cells *in vivo* in NSG mice following treatment with N1012⁺ T-cells as ascertained by bioluminescence imaging (BLI). BLI average total flux (photons/second) per treatment group is shown in Figure 12A, and BLI total flux (photons/second) per individual mouse is shown in Figure 12B. To confirm T-cell persistence and maintenance of function, all tumour-free mice were inoculated i.p. with an additional 1×10⁶ ffLUC-tagged H226 cells, 91 days after initial tumour inoculation, the rechallenge shown as a dotted line in the graphs.

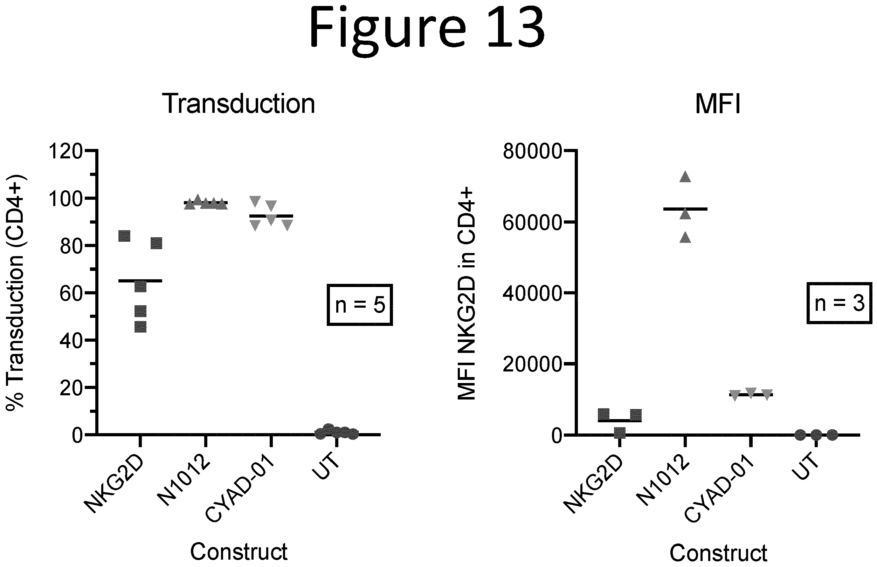

Figure 13 shows the surface expression of NKG2D, N1012 and CYAD-01 when expressed in CD4⁺ T-cells.

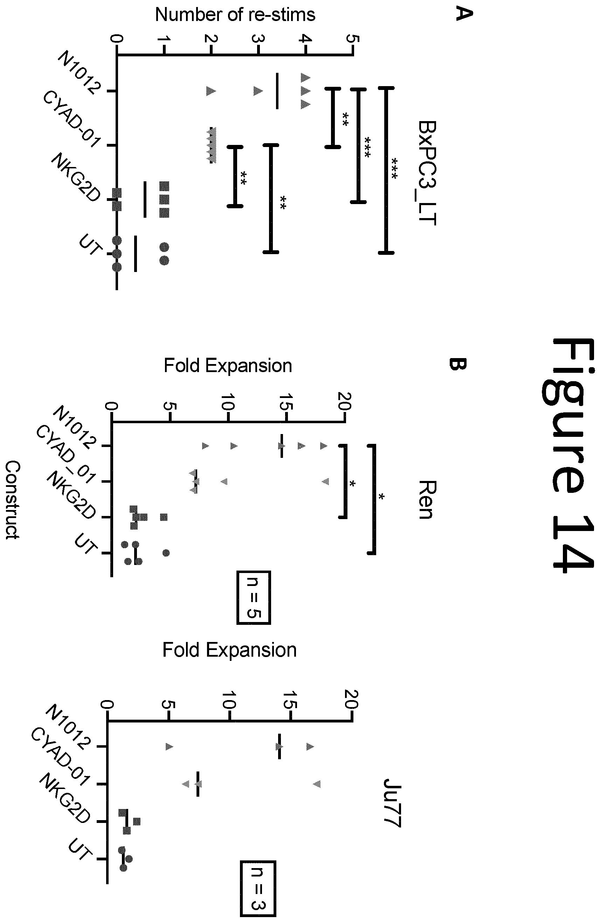

Figure 14 compares the restimulation (Figure 14A) and proliferation potential (Figure 14B) of N1012⁺ T-cells to CYAD-01 T-cells and NKG2D T-cells when co-cultured with BxPC3-LT, Ren or Ju77 cells.

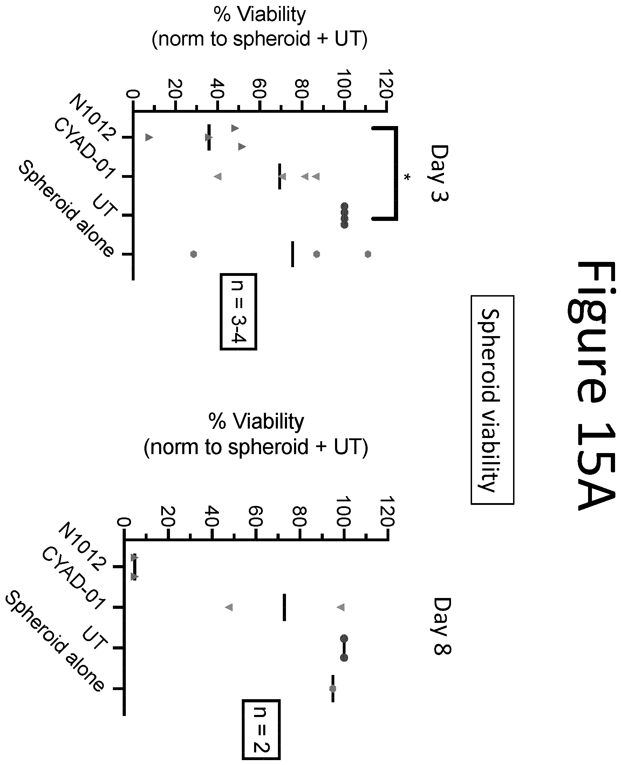

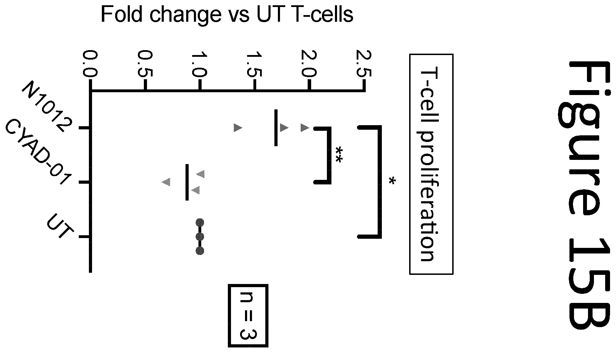

Figure 15A shows the viability of tumour spheroids following co-culture with N1012⁺, CYAD-01 or UT T-cells. Figure 15B shows the level of proliferation of the T-cells during the co-cultures with tumour spheroids.

### DETAILED DESCRIPTION

### DAP10 polypeptides and functional variants thereof

The DAP10 polypeptide used in the fusion polypeptide described herein may be mammalian, for example human. Wild-type human DAP10 is encoded by the amino acid sequence having UniProt accession no: Q9UBK5 (SEQ ID NO: 1). This is a 93aa polypeptide. The first 18aa are considered to be a signal/leader sequence, amino acids 19-48 the extracellular domain, amino acids 49-69 the transmembrane domain, and amino acids 70-93 the cytoplasmic/intracellular domain.

In one embodiment, a DAP10 polypeptide used in the fusion polypeptide of the invention comprises an amino acid sequence having at least about 80%, at least about 85%, at least about 90%, at least about 95%, at least about 97%, at least about 98% or at least about 99% sequence identity to the DAP10 polypeptide of SEQ ID NO: 1. In some embodiments, a DAP10 polypeptide used in the fusion polypeptide of the invention comprises an amino acid sequence of SEQ ID NO: 1.

In another embodiment, a functional variant DAP10 polypeptide used in the fusion polypeptide of the invention may comprise one or more (i.e. 1, 2, 3, 4, 5, 6, 7, 8, 9, 10 or more) point mutations that add, delete or substitute any of the amino acids of the amino acids of DAP10 (such as that of wild-type human DAP10 (SEQ ID NO:1)).

Truncated versions of a DAP10 polypeptide may also be used in fusion polypeptides of the invention. For example, a truncated version of DAP10 comprising only amino acids 19-93 of SEQ ID NO:1 (i.e. lacking amino acids 1-18, the signal/leader sequence) may be used in the fusion polypeptide of the invention. Such a sequence is referred to as SEQ ID NO: 2 herein. Other truncated versions may comprise amino acids 19-69 of SEQ ID NO: 1, such a sequence comprising merely the extracellular and transmembrane domains of DAP10, and referred to herein as SEQ ID NO:3. A further truncated version of DAP10 used in the invention may comprise amino acids 1-71 of SEQ ID NO: 1 (i.e. the signal/leader sequence, extracellular domain, transmembrane domain and 2 amino acids from the cytoplasmic/intracellular domain), referred to as SEQ ID NO: 4 herein. A further truncated version of DAP10 used in the invention may comprise amino acids 19-71 of SEQ ID NO: 1 (i.e. the extracellular domain, transmembrane domain and 2 amino acids from the cytoplasmic/intracellular domain), referred to as SEQ ID NO: 5 herein. A further truncated version of DAP10 used in the invention may comprise amino acids 70-93 of SEQ ID NO: 1 (i.e. the intracellular domain), referred to as SEQ ID NO: 6 herein. A yet further truncated version of DAP10 used in the invention may comprise amino acids 49-93 of SEQ ID NO: 1 (i.e. the transmembrane and cytoplasmic/intracellular domains), referred to as SEQ ID NO: 7 herein. A yet further truncated version of DAP10 used in the invention may comprise amino acids 49-69 of SEQ ID NO: 1 (i.e. the transmembrane domain), referred to as SEQ ID NO: 8 herein.

Other mutated versions or truncated versions of the DAP10 polypeptide are also suitable for use in the invention. In some embodiments such mutated versions or truncated versions that are used as functional variants of DAP10 polypeptides in the invention retain the activity of the wild type polypeptide shown in SEQ ID NO: 1.

In one embodiment, a functional variant of a DAP10 polypeptide of the invention retains at least 10% (for example 20%, 30%, 40%, 50%, 60%, 70%, 80%, 90%, 95%, 97%, 98%, 99% or more) of the activity of the wild type polypeptide shown in SEQ ID NO: 1. In one embodiment, the activity may be measured by assessment of tyrosine phosphorylation of DAP10 and/or recruitment and activation of the p85 subunit of phosphatidylinositol 3-kinase and the downstream anti-apoptotic kinase, AKT.

### DAP12 polypeptides and functional variants thereof

The DAP12 polypeptide used in the fusion polypeptide described herein may be mammalian, for example human. Wild-type human DAP12 is encoded by the amino acid sequence having UniProt accession no: 043914 (SEQ ID NO: 9). The first 21 amino acids are considered to be a signal/leader sequence, amino acids 22-40 the extracellular domain, amino acids 41-61 the transmembrane domain, and amino acids 62-113 the cytoplasmic/intracellular domain.

In one embodiment, a DAP12 polypeptide used in the fusion polypeptide of the invention comprises an amino acid sequence having at least about 80%, at least about 85%, at least about 90%, at least about 95%, at least about 97%, at least about 98% or at least about 99% sequence identity to the DAP12 polypeptide of SEQ ID NO: 9. In some embodiments, a DAP12 polypeptide used in the fusion polypeptide of the invention comprises an amino acid sequence of SEQ ID NO: 9.

In another embodiment, a functional variant DAP12 polypeptide used in the fusion polypeptide of the invention may comprise one or more (i.e. 1, 2, 3, 4, 5, 6, 7, 8, 9, 10 or more) point mutations that add, delete or substitute any of the amino acids of the amino acids of DAP12 (such as that of wild-type human DAP12 (SEQ ID NO: 9)).

Truncated versions of a DAP12 polypeptide may also be used in fusion polypeptides of the invention. For example, a truncated version of DAP12 comprising only amino acids 22-113 of SEQ ID NO: 9 (i.e. lacking amino acids 1-21, the signal/leader sequence) may be used in the fusion polypeptide of the invention. Such a sequence is referred to as SEQ ID NO: 10 herein. Other truncated versions may comprise amino acids 62-113 of SEQ ID NO: 9, such a sequence comprising merely the cytoplasmic/intracellular domain of DAP12, and referred to herein as SEQ ID NO:11. Other truncated versions may comprise amino acids 41-61 of SEQ ID NO: 9 (i.e. the transmembrane domain), referred to as SEQ ID NO: 12 herein. Another truncated version may comprise amino acids 22-61 of SEQ ID NO: 9 (i.e. the extracellular and transmembrane domains), referred to as SEQ ID NO: 13 herein.

Other mutated versions or truncated versions of the DAP12 polypeptide are also suitable for use in the invention. Mutated versions or truncated versions that are used as functional variants of DAP12 polypeptides in the invention may retain the activity of the wild type polypeptide shown in SEQ ID NO: 9.

In one embodiment, a functional variant of a DAP12 polypeptide of the invention retains at least 10% (for example 20%, 30%, 40%, 50%, 60%, 70%, 80%, 90%, 95%, 97%, 98%, 99% or more) of the activity of the wild type polypeptide shown in SEQ ID NO: 9. In one embodiment, the activity may be measured using functional assays, such as MTT and measuring cytokine secretion by ELISA.

### Polypeptides that associate with fusion polypeptides of the invention

The fusion polypeptide of the invention may associate with other polypeptides. Such association may be due to electrostatic forces, such as provided by complementary charged amino acids.

One example of such a polypeptide that may associate with a fusion polypeptide of the invention is the NKG2D polypeptide (Wu et al., 2000, J. Exp. Med., 192(7):1059-1067 and Rosen et al., 2004, J. Immunol., 173(4):2470-2478).

In one embodiment, such polypeptides may be genetically encoded as part of a contiguous chimeric construct with the gene that encodes for the fusion polypeptide of the invention. The fusion polypeptide and other polypeptide may then be separated during translation (e.g. using a ribosomal skip peptide) or by post translation cleavage (e.g. using a furin cleavage site). The fusion polypeptide and other polypeptide may therefore be joined by an optional linker. Such a linker may comprise a cleavage site to facilitate cleavage.

### NKG2D polypeptides and functional variants thereof

The NKG2D polypeptide used in a chimeric polypeptide described herein may be mammalian, for example human. Wild-type human NKG2D is encoded by the amino acid sequence having UniProt accession no: P26718 (SEQ ID NO: 14). The polypeptide is considered to comprise a cytoplasmic domain (amino acids 1-51), a transmembrane domain (amino acids 52-72) and an extracellular domain (amino acids 73-216).

In one embodiment, a NKG2D polypeptide used in the invention comprises an amino acid sequence having at least about 80%, at least about 85%, at least about 90%, at least about 95%, at least about 97%, at least about 98% or at least about 99% sequence identity to the NKG2D polypeptide of SEQ ID NO: 14. In some embodiments, a NKG2D polypeptide used in the invention comprises an amino acid sequence of SEQ ID NO: 14.

In another embodiment, a functional variant NKG2D polypeptide used in the invention may comprise one or more (i.e. 1, 2, 3, 4, 5, 6, 7, 8, 9, 10 or more) point mutations that add, delete or substitute any of the amino acids of the amino acids of NKG2D (such as that of wild-type human NKG2D (SEQ ID NO: 14)).

Truncated versions of a NKG2D polypeptide may also be used in polypeptides of the invention. For example, a truncated version of NKG2D comprising only amino acids 73-216 of SEQ ID NO:14 (i.e. the extracellular domain) may be used in the invention. Such a sequence is referred to as SEQ ID NO: 15 herein. Other truncated versions may comprise amino acids 82-216 of SEQ ID NO: 14, such a sequence comprising part of the extracellular domain of NKG2D, and referred to herein as SEQ ID NO:16. A further truncated version comprises amino acids 52-216 of SEQ ID NO: 14 (i.e. the transmembrane and extracellular domains), referred to as SEQ ID NO: 17 herein.

Other mutated versions or truncated versions of the NKG2D polypeptide are also suitable for use in the invention. Of course, any such mutated versions or truncated versions that are used as functional variants of NKG2D polypeptides in the invention should preferably retain the activity of the wild type polypeptide shown in SEQ ID NO: 14.

In one embodiment, a functional variant of a NKG2D polypeptide of the invention retains at least 10% (for example 20%, 30%, 40%, 50%, 60%, 70%, 80%, 90%, 95%, 97%, 98%, 99% or more) of the activity of the wild type polypeptide shown in SEQ ID NO: 14. In one embodiment, the activity may be measured using flow cytometry to confirm continued binding to NKG2D ligands and through various cell culture assays (such as MTT and ELISA) aimed at confirming target cell lysis, cytokine secretion and co-stimulation.

### Linkers

The DAP10 and DAP12 moieties described in this disclosure can be directly bonded to each other in a contiguous polypeptide chain, or may be indirectly bonded to each other through a suitable linker. The linker may be a peptide linker. Peptide linkers are commonly used in fusion polypeptides and methods for selecting or designing linkers are well-known. (See, e.g., Chen X et al., 2013, Adv. Drug Deliv. Rev. 65(10):135701369 and Wriggers W et al., 2005, Biopolymers 80:736-746.) Linkers may also be used to join the fusion polypeptide of the invention to another polypeptide (such as a NKG2D polypeptide) in a chimeric construct as described above.

Peptide linkers generally are categorized as i) flexible linkers, ii) helix forming linkers, and iii) cleavable linkers, and examples of each type are known in the art. In one example, a flexible linker is included in the fusion polypeptides described herein. Flexible linkers may contain a majority of amino acids that are sterically unhindered, such as glycine and alanine. The hydrophilic amino acid Ser is also conventionally used in flexible linkers. Examples of flexible linkers include, without limitation: polyglycines (e.g., (Gly)₄ and (Gly)₅), polyalanines poly(Gly-Ala), and poly(Gly-Ser) (e.g., (Glyₙ-Serₙ)ₙ or (Serₙ-Glyₙ)ₙ, wherein each n is independently an integer equal to or greater than 1).

Peptide linkers can be of a suitable length. The peptide linker sequence may be at least 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, or more amino acid residues in length. For example, a peptide linker can be from about 5 to about 50 amino acids in length; from about 10 to about 40 amino acids in length; from about 15 to about 30 amino acids in length; or from about 15 to about 20 amino acids in length. Variation in peptide linker length may retain or enhance activity, giving rise to superior efficacy in activity studies. The peptide linker sequence may be comprised of naturally or non-naturally occurring amino acids, or a mixture of both naturally and non-naturally occurring amino acids.

In some aspects, the amino acids glycine and serine comprise the amino acids within the linker sequence. In certain aspects, the linker region comprises sets of glycine repeats (GSG₃)ₙ (SEQ ID NO:18), where n is a positive integer equal to or greater than 1 (for example 1 to about 20). More specifically, the linker sequence may be GSGGG (SEQ ID NO: 19). The linker sequence may be GSGG (SEQ ID NO: 20). In certain other aspects, the linker region orientation comprises sets of glycine repeats (SerGly₃)ₙ, where n is a positive integer equal to or greater than 1 (for example 1 to about 20) (SEQ ID NO: 21).

In other embodiments, a linker may contain glycine (G) and serine (S) in a random or a repeated pattern. For example, the linker can be (GGGGS)ₙ (SEQ ID NO: 22), wherein n is an integer ranging from 1 to 20, for example 1 to 4. In a particular example, n is 4 and the linker is GGGGSGGGGSGGGGSGGGGS (SEQ ID NO: 23). In another particular example, n is 3 and the linker is GGGGSGGGGSGGGGS (SEQ ID NO: 24).

In other embodiments, a linker may contain glycine (G), serine (S) and proline (P) in a random or repeated pattern. For example, the linker can be (GPPGS)ₙ, wherein n is an integer ranging from 1 to 20, for example 1-4. In a particular example, n is 1 and the linker is GPPGS (SEQ ID NO: 25).

In general, the linker is not immunogenic when administered in a patient, such as a human. Thus, linkers may be chosen such that they have low immunogenicity or are thought to have low immunogenicity.

The linkers described herein are exemplary, and the linker can include other amino acids, such as Glu and Lys, if desired. The peptide linkers may include multiple repeats of, for example, (G₃S) (SEQ ID NO: 26), (G₄S) (SEQ ID NO: 27), (GYS) (SEQ ID NO: 28), and/or (GlySer) (SEQ ID NO: 29), if desired. In certain aspects, the peptide linkers may include multiple repeats of, for example, (SG₄) (SEQ ID NO: 30), (SG₃) (SEQ ID NO: 31), (SG₂) (SEQ ID NO: 32), (SG)₂ (SEQ ID NO: 33) or (SerGly) (SEQ ID NO: 34).

In other aspects, the peptide linkers may include combinations and multiples of repeating amino acid sequence units, such as (G₃S)+(G₄S)+(GlySer) (SEQ ID NO: 26 +SEQ ID NO: 27 +SEQ ID NO: 29). In other aspects, Ser can be replaced with Ala e.g., (G₄A) (SEQ ID NO: 35) or (G₃A) (SEQ ID NO: 36). In yet other aspects, the linker comprises the motif (EAAAK)ₙ, where n is a positive integer equal to or greater than 1, for example 1 to about 20 (SEQ ID NO: 37). In certain aspects, peptide linkers may also include cleavable linkers.

The linkers may comprise further domains and/or features, such as a furin cleavage site (RRKR)(SEQ ID NO: 38), a P2A ribosomal skip peptide (ATNFSLLKQAGDVEENPGP)(SEQ ID NO: 39) and/or a T2A ribosomal skip peptide (EGRGSLLTCGDVEENPGP)(SEQ ID NO: 40). Examples of linkers comprising these domains include SGSG + a P2A ribosomal skip peptide (SGSGATNFSLLKQAGDVEENPGP)(SEQ ID NO: 41), SGSG + a T2A ribosomal skip peptide (SGSGEGRGSLLTCGDVEENPGP)(SEQ ID NO: 42), and versions also including a furin cleavage site, i.e. furin cleavage site + SGSG + a P2A ribosomal skip peptide (RRKRSGSGATNFSLLKQAGDVEENPGP) (SEQ ID NO: 43) and furin cleavage site + SGSG + a T2A ribosomal skip peptide (RRKRSGSGEGRGSLLTCGDVEENPGP) (SEQ ID NO: 44). Alternative ribosomal skip peptides that may be used in the invention include F2A (VKQTLNFDLLKLAGDVESNPGP) (SEQ ID NO: 45) and E2A (QCTNYALLKLAGDVESNPGP) (SEQ ID NO: 46).

### N-terminal sequences & C-terminal sequences

Various sequences may be attached to the N- or C-terminus of the fusion polypeptides of the invention, or to the NKG2D polypeptides disclosed herein. These may be functional, such as signal peptides, purification tags/sequences, or half-life extension moieties, or may simply comprise spacer sequences. Alternatively, they may comprise a function, such as a T-cell stimulatory function.

### Purification tags and markers

A variety of tags or markers may be attached to the N- or C-terminus of the fusion polypeptides of the invention to assist with purification. Any affinity tag may be combined with the fusion polypeptides of the invention to assist with purification. Examples of such affinity tags are a His-tag, a FLAG-tag, Arg-tag, T7-tag, Strep-tag, S-tag, aptamer-tag, V5 tag, AviTag^{™}, myc epitope tag or any combination of these tags. In one embodiment the affinity tag is a His-tag (usually comprising 5-10 histidine residues), for example a 6His tag (i.e. HHHHHH) (SEQ ID NO: 47). In another embodiment the affinity tag is a FLAG tag (i.e. DYKDDDDK) (SEQ ID NO: 48). In another embodiment, the affinity tag is an AviTag^{™} (i.e. GLNDIFEAQKIEWHE) (SEQ ID NO: 49). In another embodiment, the affinity tag is a V5 tag (GKPIPNPLLGLDST) (SEQ ID NO: 50) or (IPNPLLGLD) (SEQ ID NO: 51). In another embodiment, the affinity tag is a myc epitope tag recognised by the 9e10 antibody (EQKLISEEDL) (SEQ ID NO: 52). Various other tags for use in the invention are well known in the art.

Combinations of such affinity tags may also be used, either comprising one or more tags at the N-terminus, one or more tags at the C-terminus, or one or more tags at each of the N-terminus and the C-terminus. Examples of such combinations include a His tag (H) combined with an AviTag (A), or a His tag (H) combined with both an AviTag (A) and a FLAG tag (F). The tags may be in either orientation, thus the AviTag/His tag may have the orientation N-AH-C or N-HA-C, while the Avi/His/FLAG tag may have the orientation N-AHF-C, N-FHA-C, etc.

In one embodiment, a fusion polypeptide according to the disclosure comprises an "AHF" tag having the sequence "GLNDIFEAQKIEWHEGGHHHHHHDYKDDDDK" (SEQ ID NO: 53). In another embodiment, a fusion polypeptide according to the disclosure comprises an "FHA" tag having the sequence "DYKDDDDKHHHHHHGGGLNDIFEAQKIEWHE" (SEQ ID NO: 54).

The CD8α leader sequence (amino acids 1-21 of UniProt: P01732 or a shortened derivative comprising amino acids 1-18), is a commonly used T-cell sequence, and is referred to as SEQ ID NO: 55 herein.

### Co-stimulatory sequences

Various T-cell co-stimulatory activation sequences are known from previous work to engineer CAR-T cells. These may also be added to fusion polypeptides of the invention.

The 4-1BB endodomain (amino acids 214-255 of UniProt: Q07011) may also be used as an N- or C-terminal sequence. The 4-1BB endodomain is referred to as SEQ ID NO: 56 herein. The 4-1BB endodomain may act as a co-stimulatory domain.

The CD27 endodomain (amino acids 213-260 of UniProt: P26842) may also be used as an N- or C-terminal sequence. The CD27 endodomain is referred to as SEQ ID NO: 57 herein. The CD27 endodomain may act as a co-stimulatory domain.

The human IgG1 hinge (amino acids 218-229 of UniProt: P0DOX5) may also be used as an N- or C-terminal sequence. The human IgG1 hinge is referred to as SEQ ID NO: 58.

A truncated CD8α hinge (amino acids 138-182 of Uniprot: P01732) may also be used as an N- or C-terminal sequence. The truncated CD8a hinge is referred to as SEQ ID NO: 59.

### Exemplary constructs

The present disclosure provides the following exemplary fusion polypeptide constructs in Table 1:

**Table 1: Exemplary DAP10/DAP12 fusion polypeptide constructs**

| **Name** | **Sequence** |
|---|---|
| DAP10 (full sequence - aa1-93)-DAP12 endodomain (aa62-113) (SEQ ID NO: 60) | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYK |
| CD8_{α} leader-FLAG-DAP10 (extracellular and TM - aa19-69)-DAP12 endodomain (aa62-113) (SEQ ID NO: 61) | MALPVTALLLPLALLLHAARPDYKDDDDKQTTPGERSSLPAFYPGTSGSC SGCGSLSLPLLAGLVAADAVASLLIVGAVFYFLGRLVPRGRGAAEAATRK QRITETESPYQELQGQRSDVYSDLNTQRPYYK |
| CD8_{α} leader-FLAG-human IgG1 hinge (aa 218-229 Uniprot ref P0DOX5)-DAP10 (TM and endodomain aa 49-93)-DAP12 endodomain (aa 62-113) (SEQ ID NO: 62) | MALPVTALLLPLALLLHAARPDYKDDDDKEPKSCDKTHTCPLLAGLVAAD AVASLLIVGAVFLCARPRRSPAQEDGKVYINMPGRGYFLGRLVPRGRGAA EAATRKQRITETESPYQELQGQRSDVYSDLNTQRPYYK |
| CD8_{α} leader-truncated CD8a hinge (aa 138-182 Uniprot reference P01732)-DAP10 (TM and endodomain-aa 49-93)-DAP12 endodomain (aa 62-113) (SEQ ID NO: 63) | MALPVTALLLPLALLLHAARPDYKDDDDKTTTPAPRPPTPAPTIASQPLSLR PEACRPAAGGAVHTRGLDFACDLLAGLVAADAVASLLIVGAVFLCARPRR SPAQEDGKVYINMPGRGYFLGRLVPRGRGAAEAATRKQRITETESPYQEL QGQRSDVYSDLNTQRPYYK |

Furthermore, as mentioned above, fusion polypeptides of the invention may be expressed as a single chimeric construct with a NKG2D polypeptide, for translation-associated or post-translational cleavage. In such constructs, following expression, the translated polypeptides are cleaved to create the separate polypeptides which then self-associate to form a CAR. In one embodiment, a fusion polypeptide of the invention is cleaved from a NKG2D polypeptide. Examples of such constructs are shown in Table 2:

**Table 2: Exemplary chimeric constructs**

| **Name** | **Sequence** |
|---|---|
| DAP10 (aa1-93)-DAP12 endodomain (aa62-113)-Furin cleavage-linker-P2A-NKG2D (aa1-216) (Construct 1) (SEQ ID NO: 64) (N1012) | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYKRRKRS GSGATNFSLLKQAGDVEENPGPMGWIRGRRSRHSWEMSEFHNYNLDLK KSDFSTRWQKQRCPVVKSKCRENASPFFFCCFIAVAMGIRFIIMVAIWSA VFLNSLFNQEVQIPLTESYCGPCPKNWICYKNNCYQFFDESKNWYESQA SCMSQNASLLKVYSKEDQDLLKLVKSYHWMGLVHIPTNGSWQWEDGSI LSPNLLTIIEMQKGDCALYASSFKGYIENCSTPNTYICMQRTV |
| CD8_{α} leader-FLAG-DAP10 (aa19-69)-DAP12 (aa62-113) - Furin cleavage-linker-T2A-DAP10 (aa 1-93)-Furin cleavage-linker-P2A-NKG2D (aa1-216) (Construct 3) (SEQ ID NO: 65) | MALPVTALLLPLALLLHAARPDYKDDDDKQTTPGERSSLPAFYPGTSGSC SGCGSLSLPLLAGLVAADAVASLLIVGAVFYFLGRLVPRGRGAAEAATRK QRITETESPYQELQGQRSDVYSDLNTQRPYYKRRKRSGSGEGRGSLLTC GDVEENPGPMIHLGHILFLLLLPVAAAQTTPGERSSLPAFYPGTSGSCSGC GSLSLPLLAGLVAADAVASLLIVGAVFLCARPRRSPAQEDGKVYINMPGR GRRKRSGSGATNFSLLKQAGDVEENPGPMGWIRGRRSRHSWEMSEFH NYNLDLKKSDFSTRWQKQRCPVVKSKCRENASPFFFCCFIAVAMGIRFII MVAIWSAVFLNSLFNQEVQIPLTESYCGPCPKNWICYKNNCYQFFDESK NWYESQASCMSQNASLLKVYSKEDQDLLKLVKSYHWMGLVHIPTNGSW QWEDGSILSPNLLTIIEMQKGDCALYASSFKGYIENCSTPNTYICMQRTV |
| DAP10 (aa1-93)-DAP12 endodomain (aa62-113)-Furin | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYKRRKRS GSGATNFSLLKQAGDVEENPGPMGWIRGRRSRHSWEMSEFHNYNLDLK KSDFSTRWQKQRCPVVKSKCRENASPFFFCCFIAVAMGIRFIIMVAIWSA |
| cleavage-linker-P2A-NKG2D (aa1-216)-Furin cleavage-linker-T2A-DAP10 (aa1-71)-4-1BB endodomain (aa214-255) (Construct 8) (SEQ ID NO: 66) | VFLNSLFNQEVQIPLTESYCGPCPKNWICYKNNCYQFFDESKNWYESQA SCMSQNASLLKVYSKEDQDLLKLVKSYHWMGLVHIPTNGSWQWEDGSI LSPNLLTIIEMQKGDCALYASSFKGYIENCSTPNTYICMQRTVRRKRSGS GEGRGSLLTCGDVEENPGPMIHLGHILFLLLLPVAAAQTTPGERSSLPAFY PGTSGSCSGCGSLSLPLLAGLVAADAVASLLIVGAVFLCKRGRKKLLYIFK QPFMRPVQTTQEEDGCSCRFPEEEEGGCEL |
| DAP10 (aa1-93)-DAP12 endodomain (aa62-113)-Furin cleavage-linker-P2A-NKG2D (aa1-216)-Furin cleavage-linker-T2A-CD8α leader (aa1-21)-FLAG-DAP10 (aa19-71)-4-1BB endodomain (aa214-255) (Construct 9) (SEQ ID NO: 67) | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYKRRKRS GSGATNFSLLKQAGDVEENPGPMGWIRGRRSRHSWEMSEFHNYNLDLK KSDFSTRWQKQRCPVVKSKCRENASPFFFCCFIAVAMGIRFIIMVAIWSA VFLNSLFNQEVQIPLTESYCGPCPKNWICYKNNCYQFFDESKNWYESQA SCMSQNASLLKVYSKEDQDLLKLVKSYHWMGLVHIPTNGSWQWEDGSI LSPNLLTIIEMQKGDCALYASSFKGYIENCSTPNTYICMQRTVRRKRSGS GEGRGSLLTCGDVEENPGPMALPVTALLLPLALLLHAARPDYKDDDDKQT TPGERSSLPAFYPGTSGSCSGCGSLSLPLLAGLVAADAVASLLIVGAVFLC KRGRKKLLYIFKQPFMRPVQTTQEEDGCSCRFPEEEEGGCEL |
| DAP10 (aa1-93)-DAP12 endodomain (aa62-113)-Furin cleavage-linker-P2A-NKG2D (aa1-216)-Furin cleavage-linker-T2A-DAP10 (aa1-71)-CD27 endodomain (aa213-260) (Construct 10) (SEQ ID NO: 68) | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYKRRKRS GSGATNFSLLKQAGDVEENPGPMGWIRGRRSRHSWEMSEFHNYNLDLK KSDFSTRWQKQRCPVVKSKCRENASPFFFCCFIAVAMGIRFIIMVAIWSA VFLNSLFNQEVQIPLTESYCGPCPKNWICYKNNCYQFFDESKNWYESQA SCMSQNASLLKVYSKEDQDLLKLVKSYHWMGLVHIPTNGSWQWEDGSI LSPNLLTIIEMQKGDCALYASSFKGYIENCSTPNTYICMQRTVRRKRSGS GEGRGSLLTCGDVEENPGPMIHLGHILFLLLLPVAAAQTTPGERSSLPAFY PGTSGSCSGCGSLSLPLLAGLVAADAVASLLIVGAVFLCQRRKYRSNKGE SPVEPAEPCHYSCPREEEGSTIPIQEDYRKPEPACSP |
| DAP10 (aa1-93)-DAP12 endodomain (aa62-113)-Furin cleavage-linker-P2A-NKG2D (aa1-216)- | MIHLGHILFLLLLPVAAAQTTPGERSSLPAFYPGTSGSCSGCGSLSLPLLA GLVAADAVASLLIVGAVFLCARPRRSPAQEDGKVYINMPGRGYFLGRLVP RGRGAAEAATRKQRITETESPYQELQGQRSDVYSDLNTQRPYYKRRKRS GSGATNFSLLKQAGDVEENPGPMGWIRGRRSRHSWEMSEFHNYNLDLK KSDFSTRWQKQRCPVVKSKCRENASPFFFCCFIAVAMGIRFIIMVAIWSA VFLNSLFNQEVQIPLTESYCGPCPKNWICYKNNCYQFFDESKNWYESQA SCMSQNASLLKVYSKEDQDLLKLVKSYHWMGLVHIPTNGSWQWEDGSI LSPNLLTIIEMQKGDCALYASSFKGYIENCSTPNTYICMQRTVRRKRSGS |
| Furin cleavage-linker-T2A- CD8_{α} leader (aa1-21)-FLAG-DAP10 (aa19-71)- CD27 endodomain (aa213-260) (Construct 11) (SEQ ID NO: 69) | GEGRGSLLTCGDVEENPGPMALPVTALLLPLALLLHAARPDYKDDDDKQT TPGERSSLPAFYPGTSGSCSGCGSLSLPLLAGLVAADAVASLLIVGAVFLC QRRKYRSNKGESPVEPAEPCHYSCPREEEGSTIPIQEDYRKPEPACSP |

### Nucleic Acid molecules encoding fusion polypeptides of the disclosure

Another aspect of the invention pertains to nucleic acid molecules that encode a fusion polypeptide or chimeric construct of the invention. This may be as DNA or RNA. Unless specifically limited herein, the term encompasses nucleic acids containing known analogues of natural nucleotides that have similar properties as the reference nucleic acid and are metabolized in a manner similar to naturally occurring nucleotides. Examples of such analogs include, without limitation, phosphorothioates, phosphoramidates, methyl phosphonates, chiral-methyl phosphorates, 2-O-methyl ribonucleotides, peptide-nucleic acids (PNAs). Unless otherwise indicated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be achieved by generating sequences in which the third position of one or more selected (or all) codons is substituted with mixed-base and/or deoxyinosine residues (Batzer et al., 1991, Nucleic Acid Res. 19:5081; Ohtsuka et al., 1985, J. Biol. Chem. 260:2605-2608; and Rossolini et al., 1994, Mol. Cell. Probes 8:91-98).

Thus, the invention also provides a nucleic acid comprising a nucleotide sequence encoding the polypeptide sequence of any one or more of SEQ ID NOs: 60-69.

The invention further provides a nucleic acid comprising a nucleotide sequence having at least about 80%, at least about 85%, at least about 90%, at least about 95%, at least about 97% or at least about 99% sequence identity with a nucleic acid encoding any of SEQ ID NOS: 60-69. Sequence identity is typically measured along the full length of the reference sequence.

The invention further provides a nucleic acid comprising a nucleotide sequence having at least about 80%, at least about 85%, at least about 90%, at least about 95%, at least about 97% or at least about 99% sequence identity with any one of SEQ ID NOs: 70-79.

The invention also provides a nucleic acid comprising the nucleotide sequence of any one of SEQ ID NOs: 70-79. The invention also provides a nucleic acid consisting of the nucleotide sequence of any one of SEQ ID NOs: 70-79.

The polynucleotide sequences can be produced by de novo solid-phase DNA synthesis or by PCR mutagenesis of an existing sequence (e.g., sequences as described in the Examples below). Direct chemical synthesis of nucleic acids can be accomplished by methods known in the art, such as the phosphotriester method of Narang et al., 1979, Meth. Enzymol. 68:90; the phosphodiester method of Brown et al., 1979, Meth. Enzymol. 68:109; the diethylphosphoramidite method of Beaucage et al., 1981, Tetra. Lett., 22:1859; and the solid support method of U.S. Pat. No. 4,458,066. Introducing mutations to a polynucleotide sequence by PCR can be performed as described in, e.g., PCR Technology: Principles and Applications for DNA Amplification, H. A. Erlich (Ed.), Freeman Press, NY, N.Y., 1992; PCR Protocols: A Guide to Methods and Applications, Innis et al. (Ed.), Academic Press, San Diego, Calif, 1990; Mattila et al., 1991, Nucleic Acids Res. 19:967; and Eckert et al., 1991, PCR Methods and Applications 1:17.

### Vectors

Also provided are vectors comprising one or more nucleic acid molecules of the invention.

For expression in host cells, the nucleic acid encoding a fusion polypeptide can be present in a suitable vector and after introduction into a suitable host, the sequence can be expressed to produce the encoded fusion polypeptide according to standard cloning and expression techniques, which are known in the art (e.g., as described in Sambrook, J., Fritsh, E. F., and Maniatis, T. Molecular Cloning: A Laboratory Manual 2nd, ed., Cold Spring Harbor Laboratory, Cold Spring Harbor Laboratory Press, Cold Spring Harbor, N.Y., 1989). The invention also relates to such vectors comprising a nucleic acid sequence according to the invention.

Various expression vectors can be employed to express the polynucleotides encoding the fusion polypeptides of the invention. Both viral-based and non-viral expression vectors can be used to produce the fusion polypeptides in a host cell, such as a mammalian host cell. Non-viral vectors and systems include plasmids, episomal vectors, typically with an expression cassette for expressing a protein or RNA, and human artificial chromosomes (see, e.g., Harrington et al., 1997, Nat Genet. 15:345). For example, non-viral vectors useful for expression of the polynucleotides and polypeptides of the fusion polypeptides of the disclosure in mammalian (e.g., human) cells include pThioHis A, B and C, pcDNA3.1/His, pEBVHis A, B and C, (Invitrogen, San Diego, Calif.), MPS V vectors, and numerous other vectors known in the art for expressing other proteins. Useful viral vectors include vectors based on retroviruses, adenoviruses, adeno-associated viruses, herpes viruses, vectors based on SV40, papilloma virus, HBP Epstein Barr virus, vaccinia virus vectors and Semliki Forest virus (SFV). See, Brent et al., supra; Smith, 1995, Annu. Rev. Microbiol. 49:807; and Rosenfeld et al., 1992, Cell 68: 143. In particular, retroviral, lentiviral, adenoviral or adeno-associated viral vectors are commonly used for expression in T-cells. Examples of such vectors include the SFG retroviral expression vector (see Riviere et al., 1995, Proc. Natl. Acad. Sci. (USA) 92:6733-6737). In one embodiment a lentiviral vector is used, these include self-inactivating lentiviral vectors (so-called SIN vectors).

The choice of expression vector depends on the intended host cells in which the vector is to be expressed. Expression vectors for mammalian host cells can include expression control sequences, such as an origin of replication, a promoter, and an enhancer (see, e.g., Queen, et al., 1986, Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP polIII promoter, the constitutive MPS V promoter, the tetracycline-inducible CMV promoter (such as the human immediate-early CMV promoter), the constitutive CMV promoter, the EF1 alpha promoter, the phosphoglycerate kinase (PGK) promoter and promoter-enhancer combinations known in the art.

Cultures of transformed organisms can be expanded under non-inducing conditions without biasing the population for coding sequences whose expression products are better tolerated by the host cells. In addition to promoters, other regulatory elements may also be required or desired for efficient expression of the antibody of the disclosure or fragments thereof. These elements typically include an ATG initiation codon and adjacent ribosome binding site or other sequences. In addition, the efficiency of expression may be enhanced by the inclusion of enhancers appropriate to the cell system in use (see, e.g., Scharf et al., 1994, Results Probl. Cell Differ. 20:125; and Bittner et al., 1987, Meth. Enzymol., 153:516). For example, the SV40 enhancer or CMV enhancer may be used to increase expression in mammalian host cells.

The invention provides a cloning or expression vector comprising a nucleic acid comprising a nucleotide sequence having at least about 80%, at least about 85%, at least about 90%, at least about 95%, at least about 97% or at least about 99% sequence identity with a nucleic acid encoding any of SEQ ID NOS: 60-69. Furthermore, the invention provides a cloning or expression vector comprising a nucleic acid encoding one or more of SEQ ID NOs: 60-69. The invention provides a cloning or expression vector comprising the nucleic acid sequence of any one of SEQ ID NOs: 70-79.

### Host Cells

Host cells comprising a polypeptide of the invention, nucleic acid of the invention, vector of the invention, or combinations of either or both thereof are provided. Such cells are generally utilized for the expression of the fusion polypeptides according to the invention.

The nucleic acid or vector may be transfected into a host cell by standard techniques.

The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like.

Alternatively, the nucleic acid or vector may be delivered into the host cell by transduction. For example, a viral vector, as disclosed above, may be used for delivery of the nucleic acid or vector.

It is possible to express the fusion polypeptides of the invention in either prokaryotic or eukaryotic host cells. Representative host cells include many *E. coli* strains, mammalian cell lines, such as CHO, CHO-K1, and HEK293; insect cells, such as Sf9 cells; and yeast cells, such as S. cerevisiae and P. pastoris. In one embodiment the host cell is an immunoresponsive cell, such as a NK cell (either a primary NK cell, or a NK cell line) or a T cell (either a primary T-cell, or a T-cell line). Other types of host cells include macrophages, induced pluripotent stem cells (iPSCs), neutrophils and invariant NKT (iNKT) cells. The T-cell may be a CD4⁺ or CD8⁺ T-cell. In one embodiment the host cell is a human cell. In one embodiment, the host cell is a human T-cell. In another embodiment, the host cell is a primary human T-cell. Cell lines which may be used include the NK cell line NK-92.

Mammalian host cells for expressing the fusion polypeptides of the invention include Chinese Hamster Ovary (CHO cells) (including dhfr- CHO cells, described Urlaub and Chasin, 1980, Proc. Natl. Acad. Sci. USA 77:4216-4220 used with a DH FR selectable marker, e.g., as described in R.J. Kaufman and P.A. Sharp, 1982, Mol. Biol. 159:601-621), NSO myeloma cells, COS cells and SP2 cells. In one embodiment the host cells are CHO K1PD cells. In another embodiment the host cells are NSO1 cells. In particular, for use with NSO myeloma cells, another expression system is the GS gene expression system shown in WO 87/04462, WO 89/01036 and EP 338,841. When recombinant expression vectors encoding fusion polypeptides are introduced into mammalian host cells, the fusion polypeptides may be produced by culturing the host cells for a period of time sufficient to allow for expression of the fusion polypeptide in the host cells or secretion of the fusion polypeptide into the culture medium in which the host cells are grown. Fusion polypeptides can be recovered from the culture medium using standard protein purification methods.

### Production methods

The present disclosure also provides methods of producing immunoresponsive cells comprising a fusion polypeptide of the invention. Such a method may comprise transducing a cell with a nucleic acid or vector encoding a fusion polypeptide of the invention. The method further comprises culturing the cell, such that the fusion polypeptide is expressed and associates with a NKG2D polypeptide to form a CAR.

In one embodiment the present invention provides a method for preparing an immunoresponsive cell comprising the steps of (i) transducing a nucleic acid or vector encoding a fusion polypeptide of the invention into the immunoresponsive cell, and (ii) culturing the immunoresponsive cell such that the fusion polypeptide is expressed and associates with a NKG2D polypeptide to form a CAR.

Various methods for the culture of immunoresponsive cells are well known in the art. See, for example, Parente-Pereira AC et al. 2014, J. Biol. Methods 1(2):e7, Ghassemi S et al. 2018, Cancer Immunol Res 6(9):1100-1109, and Denman CJ et al. 2012, PLoS One 7(1): e30264.

### Compositions

The invention also provides pharmaceutical compositions comprising a fusion polypeptide, nucleic acid, vector, host cell or immunoresponsive cell as described herein. Such pharmaceutical compositions comprise a pharmaceutically or physiologically acceptable diluent and/or carrier. The carrier is generally selected to be suitable for the intended mode of administration and can include agents for modifying, maintaining, or preserving, for example, the pH, osmolarity, viscosity, clarity, colour, isotonicity, odour, sterility, stability, rate of dissolution or release, adsorption, or penetration of the composition. Typically, these carriers include aqueous or alcoholic/aqueous solutions, emulsions or suspensions, including saline and/or buffered media.

Suitable agents for inclusion in the pharmaceutical compositions include, but are not limited to, amino acids (such as glycine, glutamine, asparagine, arginine, or lysine), antimicrobials, antioxidants (such as ascorbic acid, sodium sulphite, or sodium hydrogen-sulphite), buffers (such as borate, bicarbonate, Tris-HCl, citrates, phosphates, or other organic acids), bulking agents (such as mannitol or glycine), chelating agents (such as ethylenediamine tetraacetic acid (EDTA)), complexing agents (such as caffeine, polyvinylpyrrolidone, beta-cyclodextrin, or hydroxypropyl-beta-cyclodextrin), fillers, monosaccharides, disaccharides, and other carbohydrates (such as glucose, mannose, or dextrins), proteins (such as free serum albumin, gelatin, or immunoglobulins), colouring, flavouring and diluting agents, emulsifying agents, hydrophilic polymers (such as polyvinylpyrrolidone), low molecular weight polypeptides, salt-forming counterions (such as sodium), preservatives (such as benzalkonium chloride, benzoic acid, salicylic acid, thimerosal, phenethyl alcohol, methylparaben, propylparaben, chlorhexidine, sorbic acid, or hydrogen peroxide), solvents (such as glycerin, propylene glycol, or polyethylene glycol), sugar alcohols (such as mannitol or sorbitol), suspending agents, surfactants or wetting agents (such as pluronics; PEG; sorbitan esters; polysorbates such as Polysorbate 20 or Polysorbate 80; Triton; tromethamine; lecithin; cholesterol or tyloxapal), stability enhancing agents (such as sucrose or sorbitol), tonicity enhancing agents (such as alkali metal halides, such as sodium or potassium chloride, or mannitol sorbitol), delivery vehicles, diluents, excipients and/or pharmaceutical adjuvants.

Parenteral vehicles include sodium chloride solution, Ringer's dextrose, dextrose and sodium chloride and lactated Ringer's. Suitable physiologically-acceptable thickeners such as carboxymethylcellulose, polyvinylpyrrolidone, gelatin and alginates may be included. Intravenous vehicles include fluid and nutrient replenishers and electrolyte replenishers, such as those based on Ringer's dextrose. In some cases one might include agents to adjust tonicity of the composition, for example, sugars, polyalcohols such as mannitol, sorbitol, or sodium chloride in a pharmaceutical composition. For example, in many cases it is desirable that the composition is substantially isotonic. Preservatives and other additives, such as antimicrobials, antioxidants, chelating agents and inert gases, may also be present. The precise formulation will depend on the route of administration. Additional relevant principle, methods and components for pharmaceutical formulations are well known (see, e.g., Allen, Loyd V. Ed, (2012) Remington's Pharmaceutical Sciences, 22nd Edition).

A pharmaceutical composition of the present disclosure can be administered by one or more routes of administration using one or more of a variety of methods known in the art. As will be appreciated by the skilled artisan, the route and/or mode of administration will vary depending upon the desired results. Routes of administration for pharmaceutical compositions of the disclosure include intravenous, intramuscular, intradermal, intraperitoneal, subcutaneous, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intra-sternal injection and infusion. In one embodiment, the pharmaceutical composition is administered intratumourally. When parenteral administration is contemplated, the pharmaceutical compositions are usually in the form of a sterile, pyrogen-free, parenterally acceptable composition. A particularly suitable vehicle for parenteral injection is a sterile, isotonic solution, properly preserved. The pharmaceutical composition can be in the form of a lyophilizate, such as a lyophilized cake.

Alternatively, the pharmaceutical composition described herein can be administered by a nonparenteral route, such as a topical, epidermal or mucosal route of administration, for example, intranasally, orally, vaginally, rectally, sublingually or topically.

In certain embodiments, the pharmaceutical composition is for subcutaneous administration. Suitable formulation components and methods for subcutaneous administration of polypeptide therapeutics (e.g., antibodies, fusion polypeptides and the like) are known in the art, see, for example, US2011/0044977, US8465739 and US8476239. Typically, the pharmaceutical compositions for subcutaneous administration contain suitable stabilizers (e.g, amino acids, such as methionine, and or saccharides such as sucrose), buffering agents and tonicifying agents.

Typically, in cell therapy, the composition comprising the host cell is administered to the patient by intravenous infusion.

### Uses and Methods

The fusion polypeptides, nucleic acids, vectors, host cells or pharmaceutical compositions of the disclosure can be administered to a subject and may be used in the treatment of disease, prophylaxis and/or for delaying the onset of disease symptoms. Any references herein to methods of treatment by therapy or surgery or *in vivo* diagnostic methods are to be interpreted as references to compounds, pharmaceutical compositions and medicaments for use in those methods.

Thus, the invention provides a fusion polypeptide, nucleic acid, vector, host cell, immunoresponsive cell or pharmaceutical composition of the invention for use in the treatment of a pathological disorder. The disclosure further provides a fusion polypeptide, nucleic acid, vector, host cell or pharmaceutical composition of the disclosure for use in the treatment of a pathological disorder. The disclosure also provides the use of a fusion polypeptide, nucleic acid, vector, host cell or pharmaceutical composition of the disclosure in the manufacture of a medicament for the treatment of a pathological disorder. The disclosure further provides a method of treating a patient suffering from a pathological disorder comprising administering a therapeutically effective amount of a fusion polypeptide, nucleic acid, vector, host cell or pharmaceutical composition of the disclosure to said patient.

As used herein, the term "pathological disorder" includes cancer, including but not limited to, a solid tumour cancer, a soft tissue tumour, a metastatic lesion and a haematological cancer. For example, the cancer can be liver cancer, lung cancer, breast cancer, prostate cancer, lymphoid cancer, colon cancer, renal cancer, bone cancer, pancreatic cancer, skin cancer, cancer of the head or neck, cutaneous or intraocular malignant melanoma, uterine cancer, ovarian cancer, rectal cancer, cancer of the anal region, stomach cancer, testicular cancer, uterine cancer, carcinoma of the fallopian tubes, carcinoma of the endometrium, carcinoma of the cervix, carcinoma of the vagina, carcinoma of the vulva, Hodgkin's Disease, non-Hodgkin's lymphoma, cancer of the oesophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukaemias including acute myeloid leukaemia, chronic myeloid leukaemia, acute lymphoblastic leukaemia, chronic lymphocytic leukaemia, solid tumours of childhood, lymphocytic lymphoma, cancer of the bladder, cancer of the kidney or ureter, carcinoma of the renal pelvis, neoplasm of the central nervous system (CNS), primary CNS lymphoma, tumour angiogenesis, spinal axis tumour, brain stem glioma, pituitary adenoma, Kaposi's sarcoma, epidermoid cancer, squamous cell cancer, T-cell lymphoma, myelodysplastic syndrome (MDS), chronic myelogenous leukaemia-chronic phase (CMLCP), diffuse large B-cell lymphoma (DLBCL), cutaneous T-cell lymphoma (CTCL), peripheral T-cell lymphoma (PTCL), hepatocellular carcinoma (HCC), gastrointestinal stromal tumours (GIST), non-small cell lung carcinoma (NSCLC), squamous cell carcinoma of the head and neck (SCCHN), environmentally induced cancers including those induced by asbestos, and combinations of said cancers. In particular, the cancer can be breast cancer, such as an oestrogen receptor-positive (ERpos) breast cancer and/or a metastatic form of breast cancer.

In one embodiment the cancer is a solid tumour cancer. In one embodiment, treatment of the pathological disorder involves targeting of non-tumour cells, such as tumour-associated stromal cells. Example types of such tumour-associated stromal cells include pancreatic stromal cells. Other types of non-tumour cells that may be targeted include macrophages, regulatory T-cells and myeloid-derived suppressor cells.

In one embodiment, the patient has been pre-treated with a chemotherapeutic agent.

In one embodiment, the administration of host cells to the patient results in a decrease in tumour size of 10%, 20%, 30%, 40%, 50%, 60%, 70%, 80%, 90%, or even 100%, when compared to an untreated tumour.

The amount of host cells administered to the patent should take into account the route of administration, the cancer being treated, the weight of the patient and/or the age of the patient. In general, about 1 × 10⁶ to about 1 × 10¹¹ cells are administered to the patient. In one embodiment, about 1 × 10⁷ to about 1 × 10¹⁰ cells, or about 1 × 10⁸ to about 1 × 10⁹ cells are administered to the patient.

### General

Sequence identity can be determined by standard methods that are commonly used to compare the similarity in position of the amino acids of two polypeptides. Using a computer program such as BLAST, FASTA or Clustal Omega, two polypeptides are aligned for optimal matching of their respective amino acids (either along the full length of one or both sequences or along a pre-determined portion of one or both sequences). The programs provide a default opening penalty and a default gap penalty, and a scoring matrix such as PAM 250 [a standard scoring matrix; see Dayhoff et al., in Atlas of Protein Sequence and Structure, vol. 5, supp. 3 (1978)] can be used in conjunction with the computer program. For example, the percent identity can then be calculated as: the total number of identical matches multiplied by 100 and then divided by the sum of the length of the longer sequence within the matched span and the number of gaps introduced into the longer sequences in order to align the two sequences.

Throughout the description and claims of this specification, the words "comprise" and "contain" and variations of the words, for example "comprising" and "comprises", mean "including but not limited to", and do not exclude other components, integers or steps. Moreover, the singular encompasses the plural unless the context otherwise requires: in particular, where the indefinite article is used, the specification is to be understood as contemplating plurality as well as singularity, unless the context requires otherwise.

The term "about" in relation to a numerical value x means, for example, x±5%.

### EXAMPLES

### General Methods

### T-cell Isolation and Retroviral Transduction

Peripheral blood mononuclear cells (PBMCs) were isolated from blood samples from healthy volunteers through density-mediated centrifugation. T-cells were activated using anti-CD3 and anti-CD28 coated paramagnetic beads at a 1:2 T-cell:bead ratio for 48 hours. 1×10⁶ T-cells were plated onto a retronectin-coated plate that had been pre-treated with 3mL retroviral supernatant. Each well was subsequently treated with 3mL fresh viral supernatant and 100IU/mL IL-2. Retroviral transduction was performed with viral particles produced by stable gibbon ape leukaemia virus (GALV)-pseudotyped 293TVec stable packaging cells. Thereafter, T-cells were fed with 100IU/mL in RPMI1640 media + 5% normal human AB serum, with fresh media and IL-2 (100IU/mL) provided thrice weekly.

### Flow Cytometry

T-cell transduction and transfection of 293T cells was assessed flow cytometry, making comparison where indicated with an appropriate isotype control. To assess the expression of the NKG2D-based constructs, cells were stained with mouse anti-human CD4-FITC, mouse anti-human NKG2D-PE and mouse anti-human CD8-APC and compensated appropriately. Due to high levels of endogenous NKG2D expression in CD8⁺ T-cells, transduction efficiency was compared against NKG2D expression in untransduced CD4⁺ T-cells. Expression of the panErbB-specific T4 and TMY CARs was assessed using biotinylated goat anti-human EGF, followed by PE-conjugated streptavidin. Transduction efficiency was calculated by comparison with N1012⁺ T-cells stained using the same reagents. Prior to use, the transduction efficiency was normalised between constructs by spiking in the requisite proportion of untransduced T-cells. This ensured that all conditions were identical for the total number of CAR⁺ T-cells and overall T-cell concentration.

To perform intracellular staining, transfected 293T cells were fixed in 4% formaldehyde for 10 minutes at room temperature before washing twice in permeabilisation solution (PBS + 0.5% BSA + 0.1% saponin). The cells were subsequently stained with 500ng PE-conjugated anti-human NKG2D, or an appropriate isotype control, in the presence of 100uL permeabilisation solution. Cells were further washed twice in permeabilisation solution prior to analysis by flow cytometry.

### Dose response assays

1×10⁴ tumour cells were plated per well (100µL) of a 96-well plate and incubated at 37°C and 5% CO₂ overnight. Twenty-four hours later T-cells were added at log2 CAR T-cell:tumour cell ratios ranging from 1:1 to 1:64. After 72 hours, the T-cells were removed and 100µL MTT solution (500µg/mL) added, before the plates were incubated at 37°C and 5% CO₂ for approximately 1 hour. Following removal of the MTT solution, the resulting formazan crystals were solubilised in DMSO (100µL/well) and the absorbance measured at 560nm. Tumour cell viability was calculated as follows: (Absorbance of monolayer with T-cells/absorbance of monolayer without T-cells)*100.

### Re-stimulation assays

1×10⁵ tumour cells were plated in triplicate wells of a 24-well plate and incubated for 24 hours at 37°C and 5% CO₂. Twenty-four hours later, 1mL containing 1×10⁵ CAR⁺ T-cells were added per well. After 72 hours, the T-cells were gently removed and the well was washed with 1mL PBS. Following removal of the PBS, 1mL of MTT (at a final concentration of 500µg/mL) was added to each well and the plate incubated at 37°C and 5% CO₂ for approximately 1 hour. Absorbance was measured in the appropriate wells at 560nm and tumour cell viability calculated as detailed in the 'dose response' section. A re-stimulation was considered successful if the tumour cell viability was measured as less than 50%.

The T-cells that had been removed from the plate were centrifuged at 400xg for 5 minutes and the supernatant removed. The pellet was re-suspended in 3.2mL R5 media and 1mL added to each well of fresh tumour monolayer (1×10⁵ tumour cells per well of a 24-well plate) in triplicate. Total T-cell number was assessed by trypan blue exclusion of a small aliquot of the remaining 200µL.

### ELISA

Secretion of IFN-γ and IL-2 by T-cells were assessed in supernatant aliquots removed 24 hours after the initiation of co-culture using Duo-set and Ready-Steady-Go ELISA kits respectively.

### Tumour spheroid generation

To generate tumour spheroids, 1×10³ tumour cells and 1×10³ PS1 stellate cells were added per well of an ultra-low affinity 96-well plate and incubated for 72 hours at 37°C and 5% CO₂. Spheroid generation was confirmed by standard light microscope visualisation.

### In vivo

1×10⁵ firefly luciferase (ffLUC)-tagged BxPC3 cells were injected into the intraperitoneal cavity of NSG mice. Twelve days after tumour inoculation, mice (n = 5 per group) were treated intraperitoneally with either PBS, 4×10⁶ (N1012⁺ (N1012 (lo)), T4⁺ or TMY⁺ CAR T-cells) or 1×10⁷ (N1012 (hi) or NKG2D) CAR⁺ T-cells. Alternatively, NSG mice were inoculated i.p. with 1×10⁶ ffLUC-tagged H226 malignant mesothelioma cells. Eight days after tumour inoculation, mice were treated with either PBS, or 4×10⁶ N1012⁺ T-cells. As a control, one group of mice were treated with 4×10⁶ T-cells expressing NKG2D alone.

Tumour growth was monitored by BLI, with all data presented as total flux (photons/second) or average total flux (photons/second) per treatment. Mice were monitored closely and weighed three times per week for signs of ill health.

### Example 1: Expression of NKG2D and DAP10/12 fusion protein in 293T cells

293T cells were transfected with the SFG retroviral plasmid backbone containing either the DAP10/12 fusion protein N1012 or NKG2D expression cassette. N1012 (SEQ ID NO: 64) comprises a complex comprising an exogenous human NKG2D protein and fusion exogenous DAP10/12 homodimers according to the invention. The N1012 plasmid comprises SEQ ID NO: 74, which encodes SEQ ID NO: 64. The surface expression of NKG2D was assessed 72 hours later by flow cytometry. Whereas NKG2D expression was readily detected at the surface of 293T cells transfected with the N1012 plasmid, no NKG2D expression was detected at the surface of those transfected with the plasmid encoding the control NKG2D (Figure 2, top panel). Given that NKG2D surface expression is dependent upon the expression of DAP10, the lack of NKG2D surface expression could be explained by the absence of DAP10 co-expression within the NKG2D plasmid. To confirm the lack of surface NKG2D expression in the NKG2D-transfected 293T cells was not due to poor transfection, intracellular staining for the presence of NKG2D was undertaken. Critically, intracellular expression of NKG2D was observed in 293T cells transfected with either the N1012 or the NKG2D plasmid (Figure 2, bottom panel). This demonstrates the successful expression of NKG2D from both constructs and also confirms the requirement of DAP10 co-expression to achieve surface expression of NKG2D.

### Example 2: Expression of N1012 and NKG2D in primary human T-cells

Primary human T-cells were activated with paramagnetic beads coated with anti-human CD3 and anti-human CD28 antibodies. Forty-eight hours after activation, T-cells were engineered by retroviral transduction to express N1012 or NKG2D. Surface expression of NKG2D was assessed by flow cytometry, co-staining for CD4 and CD8 expression. The percentage expression (Figure 3A) and median fluorescence intensity (MFI, Figure 3B) of NKG2D was compared against untransduced T-cells. Due to endogenous expression of NKG2D in CD8⁺ T-cells, data are gated on CD4⁺ T-cells. As shown, both NKG2D and N1012 constructs are reproducibly expressed at high levels at the surface of primary human T-cells, in contrast to either UT T-cells, or T-cells expressing a control CAR (Figure 4).

### Example 3: Assessment of target cell destruction and recognition by N1012 T-cells

To assess cytolytic capacity, N1012⁺ T-cells were co-cultured with eleven different human tumour cell lines, representing 5 different tumour types (mesothelioma, ovarian cancer, squamous cell carcinoma of the head and neck, pancreatic cancer and breast cancer), or with tumour-associated stromal cells (PS1) at varying E:T ratios. After 72 hours, the T-cells were removed and MTT assay performed to assess tumour cell viability. Whereas a minimal reduction in tumour viability was observed when target cells were co-cultured with either UT T-cells, or those expressing NKG2D, N1012⁺ T-cells demonstrated potent lysis of all target cell lines, even at low E:T ratios (Figure 5 and 7A and B). Analysis of co-culture supernatants by ELISA demonstrated substantial secretion of both Interferon-γ (IFN-y) and Interleukin-2 (IL-2) by N1012⁺ T-cells, but not by either UT T-cells or those expressing the control NKG2D construct (Figure 6A and B). These data demonstrate the ability of N1012⁺ T-cells to recognise and lyse a broad variety of tumour types, including tumour-associated stromal cells.

### Example 4: Exemplification of tumour cell and stromal cell co-cultures destruction by N1012 T-cells

To determine whether N1012⁺ T-cells retained the ability to lyse tumour cells when grown in the presence of stromal cells, they were co-cultured with a monolayer containing both tumour and stromal cells. To achieve this, 5×10⁴ tumour cells and 5×10⁴ PS1 stellate cells were mixed and plated into triplicate wells of a 24-well plate. Twenty-four hours later, 1×10⁵ T-cells were added and the plates incubated for 72 hours. The T-cells were subsequently removed and the viability of the tumour cell and stromal cell monolayer was assessed by MTT assay as detailed in Example 3. Whereas potent lysis of both tumour and stromal cells was observed with N1012⁺ T-cells, a minimal reduction in target cell viability was observed when co-cultured with either NKG2D or UT T-cells (Figure 7C-D). Both N1012⁺ T-cells and those expressing the A2028z CAR mediated equivalent lysis of BxPC3_LT-cells grown in isolation. The a2028zCAR is a pCAR that targets the αvβ6 integrin. The targeting moiety of this CAR is composed of a VPI-derived A20FMDV2 20mer peptide (SEQ ID NO: 80) that binds to the αvβ6 integrin. This was placed downstream of a CD124 signal peptide (aa1-25 of Uniprot reference P24394, SEQ ID NO: 81). The targeting moiety is fused via a AAA linker to a partial extracellular domain, the transmembrane domain and the intracellular domain of CD28 (aa114-220, Uniprot reference P10747, SEQ ID NO: 82), in which the B7 binding residues of CD28 (aa117-122) have been replaced with aa410-419 of human c-myc (Uniprot reference P01106, SEQ ID NO: 52). This is fused in frame with aa52-164 of CD247 (Uniprot reference P20963, SEQ ID NO: 83).

In contrast, whereas N1012⁺ T-cells maintained potent lysis of monolayers comprising both BxPC3_LT tumour cells and PS1 stellate cells, the efficacy (Figure 7E-F) and cytokine secretion (Figure 7G-H) by A2028z T-cells was substantially reduced.

### Example 5: Exemplification of tumour spheroid destruction by N1012 T-cells

To assess the ability of N1012⁺ T-cells to mediate target cell lysis within a 3D system, they were co-cultured with tumour spheroids. Following spheroid generation, 6×10³ CellTracker Violet-labelled T-cells were added per well. Tumour cell and stellate cell viability was assessed after 72 hours and 192 hours using fluorescent microscopy, by measuring GFP and RFP, respectively. Quantification of the GFP and RFP signals was undertaken using Image J software and expressed as a percentage of the fluorescence readings from spheroids grown in the absence of T-cells. Potent lysis of the spheroids was observed with N1012⁺ T-cells, but not with either NKG2D or UT T-cells (Figure 7I-J). Furthermore, only N1012⁺ T-cells demonstrated secretion of IFN-y (Figure 7K).

Spheroid viability and T-cell proliferation were alternatively assessed using flow cytometry. To achieve this, the spheroids were removed from the plate, either 72 or 192 hours after T-cell addition and placed into a flow cytometry tube, with up to five spheroids treated with the same CAR T-cells added to the same tube. Spheroid disaggregation was achieved using Accutase solution and vigorous re-suspension through a pipette tip. The resulting single cell suspension was washed in RPMI1640 media + 5% normal human AB serum and subsequently re-suspended in PBS containing counting beads. An equal number of counting beads were acquired per tube and the resulting number of tumour cells (as assessed by GFP and RFP fluorescence) and T-cells (as assessed by CellTracker Violet fluorescence) determined. The data are shown as a percentage of the sum of GFP and RFP cells present in spheroids grown in the absence of T-cells.

### Example 6: Exemplification of serial target recognition by N1012 T-cells

To assess the ability of N1012⁺ T-cells to undertake serial lysis of target cells ('re-stimulation'), they were co-cultured with fresh monolayer twice weekly until monolayer destruction was not observed. Whereas UT or NKG2D⁺ T-cells mediated minimal target cell destruction and displayed no evidence of proliferation, N1012⁺ T-cells mediated potent lysis through multiple rounds of re-stimulation (Figures 8A-B). Target cell destruction was also associated with substantial proliferation and expansion of N1012⁺ T-cells (Figures 8C-D).

When compared to the CYAD-01 NKG2D CAR and control T-cells, N1012⁺ T-cells underwent significantly more rounds of re-stimulation upon BxPC3_LT-cells (Figure 14A). Furthermore, N1012⁺ T-cells also demonstrated substantially greater proliferation than CYAD-01 T-cells or controls (Figure 14B).

### Example 7: Efficacy of N1012 T-cells in in vivo models of pancreatic cancer

To determine the ability of N1012⁺ T-cells to target tumour cells *in vivo,* T-cells expressing N1012, NKG2D or two different iterations of a pan-ErbB targeting CAR (T4 and TMY) were generated. Expression of the various constructs within primary human T-cells (Figure 9A) and efficacy of the T-cells cells was demonstrated *in vitro* against three cell lines after a 72 hour co-culture at a 1:1 ratio (Figure 9B). To assess function *in vivo,* intraperitoneal firefly luciferase (ffLUC)-tagged BxPC3 tumours were established for 12 days in NSG mice. Tumour-bearing mice were treated intraperitoneally with either PBS, 4×10⁶ (N1012 (lo), T4 or TMY) T-cells, or 1×10⁷ (N1012 (hi) or NKG2D) transduced T-cells. Tumour growth was measured weekly by bioluminescence imaging, with mice weighed thrice weekly. The data are presented as both average total flux (photons/second) per treatment group (Figure 10A), or total flux (photons/second) per individual mouse (Figure 10B). Tumour burden in mice receiving NKG2D⁺, T4⁺ or TMY⁺ T-cells was identical to those receiving PBS, suggesting a lack of efficacy. In contrast, tumour was completely eradicated in 2/5 mice and 4/5 mice treated with either N1012 (lo) or N1012 (hi) respectively. These mice remained tumour free 76 days after T-cell administration. No evidence of toxicity was observed when assessed by measurement of percentage change in body weight (Figure 10C).

To determine whether N1012⁺ T-cells could engraft within NSG mice and provide immunological memory, those mice that had completely rejected tumour received a second inoculation of 1×10⁵ ffLUC-tagged BxPC3 cells into the peritoneal cavity 88 days after initial tumour inoculation (76 days post T-cell infusion). Whilst an increase in luminescence was observed by BLI 24 hours after tumour re-challenge, 4/5 mice subsequently demonstrated a substantial reduction in tumour size, thus indicating re-activation of the N1012⁺ T-cells (Figure 10D). The experiment was ended after 145 days and a survival curve generated, which demonstrated the potent anti-tumour efficacy of the N1012 T-cells (Figure 10E).

To further confirm the efficacy of N1012⁺ T-cells against a pancreatic cancer model *in vivo,* a repeat experiment was undertaken. 1×10⁷ CAR⁺ or control untransduced T-cells were injected i.p. into NSG mice twelve days after inoculation with 1×10⁵ ffLUC-tagged BxPC3 cells. Tumour growth was monitored weekly by bioluminescent imaging and the data are presented as both average total flux (photons/second) per treatment group (Figure 11A), and total flux (photons/second) per individual mouse (Figure 11B). Significant and sustained tumour regression was once again observed in mice treated with N1012⁺ T-cells. Indeed, tumour was completely eradicated in 5/6 mice treated with N1012⁺ T-cells. In contrast, the kinetics of tumour growth in mice treated with UT T-cells were identical to those receiving PBS. These data confirm that tumour eradication was N1012⁺-specific.

To investigate the potential formation of memory T-cells, mice that were tumour free at day 41 (29 days after T-cell infusion) were re-challenged i.p. with a fresh bolus of 1×10⁵ ffLUC-tagged BxPC3 cells. Mice were imaged on day 42 to confirm tumour take. Subsequent imaging demonstrated that 5/5 re-challenged mice reduced tumour burden below detection, with 3/5 mice demonstrating long term tumour control (Figure 11B). These data suggest that N1012 CAR T-cells are able to form memory and re-activate in response to re-emergence of the target.

### Example 8: Efficacy of N1012 T-cells in an in vivo model of malignant mesothelioma

To confirm the efficacy of N1012 in another *in vivo* model, NSG mice were inoculated i.p. with 1×10⁶ ffLUC-tagged H226 malignant mesothelioma cells. Eight days after tumour inoculation, mice were treated with either PBS, or 4×10⁶ N1012⁺ T-cells. As a control, one group of mice were treated with 4×10⁶ T-cells expressing NKG2D alone. Tumour growth was monitored weekly by bioluminescent imaging and the data are presented as average total flux (photons/second) per treatment (Figure 12A) and as total flux (photons/second) per individual mouse (Figure 12B). Whereas consistent tumour growth was observed in the mice that received PBS, 100% tumour eradication was observed in mice receiving N1012⁺ T-cells.

To confirm T-cell persistence and maintenance of function, all tumour-free mice were inoculated i.p. with an additional 1×10⁶ ffLUC-tagged H226 cells, 91 days after initial tumour inoculation. Tumour take was confirmed in all mice after 24 hours by bioluminescent imaging. All re-challenged mice rejected the tumour, confirming persistence of the N1012⁺ T-cells and the ability of these T-cells to mediate long-term tumour control.

### Example 9: Comparison of N1012 T-cells to CYAD-01 T-cells

The restimulation and proliferation potential of N1012 T-cells was compared to CYAD-01 T-cells. As noted previously, the CYAD-01 CAR consists of a fusion of NKG2D to CD3ζ (Zhang et al, 2005, Blood 106: 1544-1551). Although nominally a first-generation CAR, it associates with endogenous DAP10 in T-cells, meaning that both signals 1 and 2 are provided. This CAR is currently undergoing clinical development by Celyad S.A. as CYAD-01, and so is provided in these examples for the purposes of comparison only.

Surface expression of CYAD-01 was confirmed in primary human T-cells when assessed by flow cytometry (Figure 13).

Briefly, N1012 or CYAD-01 T-cells were co-cultured with fresh monolayer twice weekly until monolayer destruction was not observed. To achieve this, 1×10⁵ tumour cells were plated in triplicate wells of a 24-well plate and incubated for 24 hours at 37°C and 5% CO2. Twenty-four hours later, 1×10⁵ CAR+ T-cells were added per well at a final concentration of 1×10⁵ CAR+/mL. After 72 hours, the T-cells were gently removed and the well was washed with 1mL PBS. Following removal of the PBS, 1mL of MTT (at a final concentration of 500µg/mL) was added to each well and the plate incubated at 37°C and 5% CO₂ for approximately 1 hour. The plate was read and tumour cell viability calculated as detailed above. A re-stimulation was considered successful if the tumour cell viability was measured as less than 50%.

To investigate T-cell proliferation in response to target cell recognition, the T-cells that had been removed from the plate were spinoculated at 400xg for 5 minutes and the supernatant removed. The pellet was re-suspended in 3.2mL R5 media and 1mL added to each well of fresh tumour monolayer in triplicate. Total T-cell number was assessed by trypan blue exclusion of a small aliquot of the remaining 200µL.

When compared to the CYAD-01, NKG2D CAR and control T-cells, N1012⁺ T-cells underwent significantly more rounds of re-stimulation upon BxPC3_LT-cells (Figure 14A). Furthermore, N1012 T-cells also demonstrated substantially greater proliferation than CYAD-01 T-cells or controls (Figure 14B).

When co-cultured with tumour spheroids, a significant reduction in spheroid viability was observed with N1012⁺ T-cells, but not with UT control T-cells, nor with those expressing the functional CYAD-01 CAR (Figure 15A). N1012 T-cells also demonstrated significant proliferation compared to either UT or CYAD-01 T-cells (Figure 15B).

### SEQUENCE LISTING

SEQ ID NO: 1 (human DAP10 full sequence) 
MIHLGHILFL LLLPVAAAQT TPGERSSLPA FYPGTSGSCS GCGSLSLPLL AGLVAADAVA
SLLIVGAVFL CARPRRSPAQ EDGKVYINMP GRG
SEQ ID NO: 2 (DAP10 aa19-93 - lacking leader sequence) 
QTTPGERSSL PAFYPGTSGS CSGCGSLSLP LLAGLVAADA VASLLIVGAV FLCARPRRSP
AQEDGKVYIN MPGRG
**SEQ ID NO: 3** (DAP10 aa19-69 - extracellular/transmembrane domain)
   QTTPGERSSL PAFYPGTSGS CSGCGSLSLP LLAGLVAADA VASLLIVGAV F
SEQ ID NO: 4 (DAP10 aa1-71) 
MIHLGHILFL LLLPVAAAQT TPGERSSLPA FYPGTSGSCS GCGSLSLPLL AGLVAADAVA
SLLIVGAVFL C
**SEQ ID NO: 5** (DAP10 aa19-71)
   QTTPGERSSL PAFYPGTSGS CSGCGSLSLP LLAGLVAADA VASLLIVGAV FLC
SEQ ID NO: 6 (DAP10 aa70-93 - intracellular domain)
   LCARPRRSPA QEDGKVYINM PGRG
SEQ ID NO: 7 (DAP10 aa49-93 - transmembrane and intracellular domain)
   LLAGLVAADA VASLLIVGAV FLCARPRRSP AQEDGKVYIN MPGRG
SEQ ID NO: 8 (DAP10 aa49-69 - transmembrane domain)
   LLAGLVAADA VASLLIVGAV F
SEQ ID NO: 9 (human DAP12 full sequence) 
MGGLEPCSRL LLLPLLLAVS GLRPVQAQAQ SDCSCSTVSP GVLAGIVMGD LVLTVLIALA
VYFLGRLVPR GRGAAEAATR KQRITETESP YQELQGQRSD VYSDLNTQRP YYK
SEQ ID NO: 10 (DAP12 aa22-113 - lacking leader sequence) 
LRPVQAQAQS DCSCSTVSPG VLAGIVMGDL VLTVLIALAV YFLGRLVPRG RGAAEAATRK
QRITETESPY QELQGQRSDV YSDLNTQRPY YK
SEQ ID NO: 11 (DAP12 aa62-113 - cytoplasmic/intracellular domain)
   YFLGRLVPRG RGAAEAATRK QRITETESPY QELQGQRSDV YSDLNTQRPY YK
SEQ ID NO: 12 (DAP12 aa41-61 - transmembrane domain)
   GVLAGIVMGD LVLTVLIALA V
SEQ ID NO: 13 (DAP12 aa22-61 - extracellular and transmembrane domains)
   LRPVQAQAQS DCSCSTVSPG VLAGIVMGDL VLTVLIALAV
SEQ ID NO: 14 (human NKG2D full sequence) 
MGWIRGRRSR HSWEMSEFHN YNLDLKKSDF STRWQKQRCP VVKSKCRENA SPFFFCCFIA
VAMGIRFIIM VAIWSAVFLN SLFNQEVQIP LTESYCGPCP KNWICYKNNC YQFFDESKNW
YESQASCMSQ NASLLKVYSK EDQDLLKLVK SYHWMGLVHI PTNGSWQWED GSILSPNLLT
IIEMQKGDCA LYASSFKGYI ENCSTPNTYI CMQRTV
SEQ ID NO: 15 (human NKG2D aa73-216 - extracellular domain) 
IWSAVFLNSL FNQEVQIPLT ESYCGPCPKN WICYKNNCYQ FFDESKNWYE SQASCMSQNA
SLLKVYSKED QDLLKLVKSY HWMGLVHIPT NGSWQWEDGS ILSPNLLTII EMQKGDCALY
ASSFKGYIEN CSTPNTYICM QRTV
SEQ ID NO: 16 (human NKG2D aa82-216 - extracellular domain) 
LFNQEVQIPL TESYCGPCPK NWICYKNNCY QFFDESKNWY ESQASCMSQN ASLLKVYSKE
DQDLLKLVKS YHWMGLVHIP TNGSWQWEDG SILSPNLLTI IEMQKGDCAL YASSFKGYIE
NCSTPNTYIC MQRTV
SEQ ID NO: 17 (human NKG2D aa52-216 - transmembrane and extracellular domain) 
PFFFCCFIAV AMGIRFIIMV AIWSAVFLNS LFNQEVQIPL TESYCGPCPK NWICYKNNCY QFFDESKNWY
ESQASCMSQN ASLLKVYSKE DQDLLKLVKS YHWMGLVHIP TNGSWQWEDG SILSPNLLTI IEMQKGDCAL
YASSFKGYIE NCSTPNTYIC MQRTV
SEQ ID NO: 18 (linker)
   GSG
SEQ ID NO: 19 (linker)
   GSGGG
SEQ ID NO: 20 (linker)
   GSGG
SEQ ID NO: 21 (linker)
   SGGG
SEQ ID NO: 22 (linker)
   GGGGS
SEQ ID NO: 23 (linker)
   GGGGSGGGGSGGGGSGGGGS
SEQ ID NO: 24 (linker)
   GGGGSGGGGSGGGGS
SEQ ID NO: 25 (linker)
   GPPGS
SEQ ID NO: 26 (linker)
   GGGS
SEQ ID NO: 27 (linker)
   GGGGS
SEQ ID NO: 28 (linker)
   GYS
SEQ ID NO: 29 (linker)
   GS
SEQ ID NO: 30 (linker)
   SGGGG
SEQ ID NO: 31 (linker)
   SGGG
SEQ ID NO: 32 (linker)
   SGG
SEQ ID NO: 33 (linker)
   SGSG
SEQ ID NO: 34 (linker)
   SG
SEQ ID NO: 35 (linker)
   GGGGA
SEQ ID NO: 36 (linker)
   GGGA
SEQ ID NO: 37 (linker)
   EAAAK
SEQ ID NO: 38 (furin cleavage site)
   RRKR
SEQ ID NO:39 (P2A skip peptide)
   ATNFSLLKQAGDVEENPGP
SEQ ID NO: 40 (T2A skip peptide)
   EGRGSLLTCGDVEENPGP
SEQ ID NO: 41 (SGSG + P2A)
   SGSGATNFSLLKQAGDVEENPGP
SEQ ID NO: 42 (SGSG + T2A)
   SGSGEGRGSLLTCGDVEENPGP
SEQ ID NO: 43 (furin + SGSG + P2A)
   RRKRSGSGATNFSLLKQAGDVEENPGP
SEQ ID NO: 44 (furin + SGSG + T2A)
   RRKRSGSGEGRGSLLTCGDVEENPGP
SEQ ID NO: 45 (F2A skip peptide)
   VKQTLNFDLLKLAGDVESNPGP
SEQ ID NO: 46 (E2A skip peptide)
   QCTNYALLKLAGDVESNPGP
SEQ ID NO: 47 (His tag)
   HHHHHH
SEQ ID NO: 48 (FLAG tag)
   DYKDDDDK
SEQ ID NO: 49 (Avi tag)
   GLNDIFEAQKIEWHE
SEQ ID NO: 50 (V5 tag)
   GKPIPNPLLGLDST
SEQ ID NO: 51 (V5 tag)
   IPNPLLGLD
SEQ ID NO: 52 (Myc tag)
   EQKLISEEDL
SEQ ID NO: 53 (AHF tag)
   GLNDIFEAQKIEWHEGGHHHHHHDYKDDDDK
SEQ ID NO: 54 (FHA tag)
   DYKDDDDKHHHHHHGGGLNDIFEAQKIEWHE
SEQ ID NO: 55 (CD8α leader sequence)
   MALPVTALLL PLALLLHAAR P
SEQ ID NO: 56 (4-1BB endodomain)
   KRGRKKLLYI FKQPFMRPVQ TTQEEDGCSC RFPEEEEGGC EL
SEQ ID NO: 57 (CD27 endodomain)
   QRRKYRSNKG ESPVEPAEPC HYSCPREEEG STIPIQEDYR KPEPACSP
SEQ ID NO: 58 (human IgG1 hinge)
   EPKSCDKTHT CP
SEQ ID NO: 59 (truncated CD8α hinge)
   TTTPAPRPPT PAPTIASQPL SLRPEACRPA AGGAVHTRGL DFACD
SEQ ID NO: 60 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYK
SEQ ID NO: 61 
MALPVTALLLPLALLLHAARPDYKDDDDKQTTPGERSSLPAFYPGTSGSCSGCGSLSLPLLAGLVAADAVASLLIVG
AVFYFLGRLVPRGRGAAEAATRKQRITETESPYQELQGQRSDVYSDLNTQRPYYK
SEQ ID NO: 62 
MALPVTALLLPLALLLHAARPDYKDDDDKEPKSCDKTHTCPLLAGLVAADAVASLLIVGAVFLCARPRRSPAQEDGK
VYINMPGRGYFLGRLVPRGRGAAEAATRKQRITETESPYQELQGQRSDVYSDLNTQRPYYK
SEQ ID NO: 63 
MALPVTALLLPLALLLHAARPDYKDDDDKTTTPAPRPPTPAPTIASQPLSLRPEACRPAAGGAVHTRGLDFACDLLA
GLVAADAVASLLIVGAVFLCARPRRSPAQEDGKVYINMPGRGYFLGRLVPRGRGAAEAATRKQRITETESPYQELQG
QRSDVYSDLNTQRPYYK
SEQ ID NO: 64 (Construct 1/N1012) 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYKRRKRSGSGA
TNFSLLKQAGDVEENPGPMGWIRGRRSRHSWEMSEFHNYNLDLKKSDFSTRWQKQRCPVVKSKCRENASPFFFCCFI
AVAMGIRFIIMVAIWSAVFLNSLFNQEVQIPLTESYCGPCPKNWICYKNNCYQFFDESKNWYESQASCMSQNASLLK
VYSKEDQDLLKLVKSYHWMGLVHIPTNGSWQWEDGSILSPNLLTIIEMQKGDCALYASSFKGYIENCSTPNTYICMQ
RTV
SEQ ID NO: 65 (Construct 3) 
MALPVTALLLPLALLLHAARPDYKDDDDKQTTPGERSSLPAFYPGTSGSCSGCGSLSLPLLAGLVAADAVASLLIVG
AVFYFLGRLVPRGRGAAEAATRKQRITETESPYQELQGQRSDVYSDLNTQRPYYKRRKRSGSGEGRGSLLTCGDVEE
NPGPMIHLGHILFLLLLPVAAAQTTPGERSSLPAFYPGTSGSCSGCGSLSLPLLAGLVAADAVASLLIVGAVFLCAR
PRRSPAQEDGKVYINMPGRGRRKRSGSGATNFSLLKQAGDVEENPGPMGWIRGRRSRHSWEMSEFHNYNLDLKKSDF
STRWQKQRCPVVKSKCRENASPFFFCCFIAVAMGIRFIIMVAIWSAVFLNSLFNQEVQIPLTESYCGPCPKNWICYK
NNCYQFFDESKNWYESQASCMSQNASLLKVYSKEDQDLLKLVKSYHWMGLVHIPTNGSWQWEDGSILSPNLLTIIEM
QKGDCALYASSFKGYIENCSTPNTYICMQRTV
SEQ ID NO: 66 (Construct 8) 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYKRRKRSGSGA
TNFSLLKQAGDVEENPGPMGWIRGRRSRHSWEMSEFHNYNLDLKKSDFSTRWQKQRCPVVKSKCRENASPFFFCCFI
AVAMGIRFIIMVAIWSAVFLNSLFNQEVQIPLTESYCGPCPKNWICYKNNCYQFFDESKNWYESQASCMSQNASLLK
VYSKEDQDLLKLVKSYHWMGLVHIPTNGSWQWEDGSILSPNLLTIIEMQKGDCALYASSFKGYIENCSTPNTYICMQ
RTVRRKRSGSGEGRGSLLTCGDVEENPGPMIHLGHILFLLLLPVAAAQTTPGERSSLPAFYPGTSGSCSGCGSLSLP
LLAGLVAADAVASLLIVGAVFLCKRGRKKLLYIFKQPFMRPVQTTQEEDGCSCRFPEEEEGGCEL
SEQ ID NO: 67 (Construct 9) 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYKRRKRSGSGA
TNFSLLKQAGDVEENPGPMGWIRGRRSRHSWEMSEFHNYNLDLKKSDFSTRWQKQRCPVVKSKCRENASPFFFCCFI
AVAMGIRFIIMVAIWSAVFLNSLFNQEVQIPLTESYCGPCPKNWICYKNNCYQFFDESKNWYESQASCMSQNASLLK
VYSKEDQDLLKLVKSYHWMGLVHIPTNGSWQWEDGSILSPNLLTIIEMQKGDCALYASSFKGYIENCSTPNTYICMQ
RTVRRKRSGSGEGRGSLLTCGDVEENPGPMALPVTALLLPLALLLHAARPDYKDDDDKQTTPGERSSLPAFYPGTSG
SCSGCGSLSLPLLAGLVAADAVASLLIVGAVFLCKRGRKKLLYIFKQPFMRPVQTTQEEDGCSCRFPEEEEGGCEL
SEQ ID NO: 68 (Construct 10) 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYKRRKRSGSGA
TNFSLLKQAGDVEENPGPMGWIRGRRSRHSWEMSEFHNYNLDLKKSDFSTRWQKQRCPVVKSKCRENASPFFFCCFI
AVAMGIRFIIMVAIWSAVFLNSLFNQEVQIPLTESYCGPCPKNWICYKNNCYQFFDESKNWYESQASCMSQNASLLK
VYSKEDQDLLKLVKSYHWMGLVHIPTNGSWQWEDGSILSPNLLTIIEMQKGDCALYASSFKGYIENCSTPNTYICMQ
RTVRRKRSGSGEGRGSLLTCGDVEENPGPMIHLGHILFLLLLPVAAAQTTPGERSSLPAFYPGTSGSCSGCGSLSLP
LLAGLVAADAVASLLIVGAVFLCQRRKYRSNKGESPVEPAEPCHYSCPREEEGSTIPIQEDYRKPEPACSP
SEQ ID NO: 69 (Construct 11) 
MIHLGHILFLLLLPVAAAQTTPGERSSLPAFYPGTSGSCSGCGSLSLPLLAGLVAADAVASLLIVGAVFLCARPRRS
PAQEDGKVYINMPGRGYFLGRLVPRGRGAAEAATRKQRITETESPYQELQGQRSDVYSDLNTQRPYYKRRKRSGSGA
TNFSLLKQAGDVEENPGPMGWIRGRRSRHSWEMSEFHNYNLDLKKSDFSTRWQKQRCPVVKSKCRENASPFFFCCFI
AVAMGIRFIIMVAIWSAVFLNSLFNQEVQIPLTESYCGPCPKNWICYKNNCYQFFDESKNWYESQASCMSQNASLLK
 
VYSKEDQDLLKLVKSYHWMGLVHIPTNGSWQWEDGSILSPNLLTIIEMQKGDCALYASSFKGYIENCSTPNTYICMQ
RTVRRKRSGSGEGRGSLLTCGDVEENPGPMALPVTALLLPLALLLHAARPDYKDDDDKQTTPGERSSLPAFYPGTSG
SCSGCGSLSLPLLAGLVAADAVASLLIVGAVFLCQRRKYRSNKGESPVEPAEPCHYSCPREEEGSTIPIQEDYRKPE
PACSP
SEQ ID NO: 70 (encoding polypeptide of SEQ ID NO: 60) 
ATGATCCACCTGGGCCACATCCTGTTCCTGCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAG
SEQ ID NO: 71 (encoding polypeptide of SEQ ID NO: 61) 
ATGGCTCTGCCTGTGACAGCTCTGCTGCTGCCTCTGGCTCTGCTGCTGCACGCCGCTAGACCCGATTATAAGGACGA
CGACGACAAGCAGACCACCCCTGGCGAGCGGAGCAGCCTGCCTGCCTTCTACCCTGGCACCAGCGGCAGCTGCAGCG
GCTGCGGCAGCCTGAGCCTGCCCCTGCTGGCtGGCCTGGTGGCCGCCGACGCCGTGGCCAGCCTGCTGATCGTGGGC
GCCGTGTTCTACTTCCTGGGCAGGCTGGTGCCCAGGGGCAGGGGCGCTGCCGAGGCTGCCACCCGGAAGCAGCGGAT
CACCGAGACCGAGAGCCCCTACCAGGAGCTGCAGGGCCAGCGGAGCGACGTGTACAGCGACCTGAACACCCAGAGGC
CCTACTACAAG
SEQ ID NO: 72 (encoding polypeptide of SEQ ID NO: 62) 
ATGGCTCTGCCTGTGACAGCTCTGCTGCTGCCTCTGGCTCTGCTGCTGCACGCCGCTAGACCCGATTATAAGGACGA
CGACGACAAGGAGCCCAAGAGCTGCGACAAGACACACACATGCCCTCTTctggccggCCTGGTGGCCGCCGACGCCG
TGGCCAGCCTGCTGATCGTGGGCGCCGTGTTCCTGTGCGCCAGGCCCAGGCGGAGCCCtGCCCAGGAGGACGGCAAG
GTGTACATCAACATGCCCGGCCGGGGCTACTTCCTGGGCAGGCTGGTGCCCAGGGGCAGGGGCGCTGCCGAGGCTGC
CACCCGGAAGCAGCGGATCACCGAGACCGAGAGCCCCTACCAGGAGCTGCAGGGCCAGCGGAGCGACGTGTACAGCG
ACCTGAACACCCAGAGGCCCTACTACAAG
SEQ ID NO: 73 (encoding polypeptide of SEQ ID NO: 63) 
ATGGCTCTGCCTGTGACAGCTCTGCTGCTGCCTCTGGCTCTGCTGCTGCACGCCGCTAGACCCGATTATAAGGACGA
CGACGACAAGACCACAACACCTGCTCCTAGACCTCCCACCCCTGCTCCCACCATCGCCAGCCAGCCCCTGAGCCTGA
GACCCGAGGCCTGCAGACCCGCTGCTGGCGGCGCTGTGCATACCAGAGGCCTGGATTTCGCCTGCGACCTTctggcc
ggCCTGGTGGCCGCCGACGCCGTGGCCAGCCTGCTGATCGTGGGCGCCGTGTTCCTGTGCGCCAGGCCCAGGCGGAG
CCCtGCCCAGGAGGACGGCAAGGTGTACATCAACATGCCCGGCCGGGGCTACTTCCTGGGCAGGCTGGTGCCCAGGG
GCAGGGGCGCTGCCGAGGCTGCCACCCGGAAGCAGCGGATCACCGAGACCGAGAGCCCCTACCAGGAGCTGCAGGGC
CAGCGGAGCGACGTGTACAGCGACCTGAACACCCAGAGGCCCTACTACAAG
SEQ ID NO: 74 (encoding polypeptide of SEQ ID NO: 64/construct 1/N1012) 
ATGATCCACCTGGGCCACATCCTGTTCCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAGAGGCGGAAAAGGTCTGGGAGTGGGGCT
ACCAATTTCTCTCTCCTCAAGCAAGCCGGAGACGTTGAGGAAAACCCTGGaCCcATGGGCTGGATCCGGGGACGGAG
GAGCCGGCACAGCTGGGAGATGAGCGAGTTCCACAACTACAACCTGGACCTGAAGAAGAGCGACTTCAGCACCCGGT
GGCAGAAGCAGCGGTGCCCCGTGGTGAAGAGCAAGTGCCGGGAGAACGCCAGCCCCTTCTTCTTCTGCTGCTTCATC
GCCGTGGCtATGGGCATCCGGTTCATCATCATGGTGGCCATCTGGAGCGCCGTGTTCCTGAACAGCCTGTTCAACCA
GGAGGTGCAGATCCCCCTGACCGAGAGCTACTGCGGCCCCTGCCCCAAGAACTGGATCTGCTACAAGAACAACTGCT
ACCAGTTCTTCGACGAGAGCAAGAACTGGTACGAGAGCCAGGCCAGCTGCATGAGCCAGAACGCCAGCCTGCTGAAG
GTGTACAGCAAGGAGGACCAGGACCTGCTGAAGCTGGTGAAGAGCTACCACTGGATGGGCCTGGTGCACATCCCCAC
CAACGGCAGCTGGCAGTGGGAGGACGGCAGCATCCTGAGCCCCAACCTGCTGACCATCATCGAGATGCAGAAGGGCG
ACTGCGCCCTGTACGCCAGCAGCTTCAAGGGCTACATCGAGAACTGCAGCACCCCCAACACCTACATCTGCATGCAG
CGGACCGTG
SEQ ID NO: 75 (encoding polypeptide of SEQ ID NO: 65/construct 3) 
ATGGCTCTGCCTGTGACAGCTCTGCTGCTGCCTCTGGCTCTGCTGCTGCACGCCGCTAGACCCGATTATAAGGACGA
CGACGACAAGCAGACCACCCCTGGCGAGCGGAGCAGCCTGCCTGCCTTCTACCCTGGCACCAGCGGCAGCTGCAGCG
GCTGCGGCAGCCTGAGCCTGCCCCTGCTGGCtGGCCTGGTGGCCGCCGACGCCGTGGCCAGCCTGCTGATCGTGGGC
GCCGTGTTCTACTTCCTGGGCAGGCTGGTGCCCAGGGGCAGGGGCGCTGCCGAGGCTGCCACCCGGAAGCAGCGGAT
CACCGAGACCGAGAGCCCCTACCAGGAGCTGCAGGGCCAGCGGAGCGACGTGTACAGCGACCTGAACACCCAGAGGC
CCTACTACAAGCGGAGAAAGCGCtccGGCTCCGGCGAGGGCcgcGGCAGCCTGCTGACCTGCGGCGACGTGGAAGAG
AACCCCGGACCCATGATCCACCTGGGCCACATCCTGTTCCTGCTGCTGCCCGTGGCCGCTGCCCAAACAACACC
CGGCGAGAGATCCTCCTTGCCCGCTTTCTATCCCGGAACATCCGGAAGCTGTtccggaTGTGGATCCCTTTCTTTGc
ctttgCTTGCTGGATTGGTCGCAGCTGACGCTGTCGCTTCCCTCCTTATTGTCGGAGCTGTCTTCCTGTGCGCCAGG
CCCAGGCGGAGCCCtGCCCAGGAGGACGGCAAGGTGTACATCAACATGCCCGGCCGGGGCAGGCGGaagcgctccGG
GAGTGGGGCTACCAATTTCTCTCTCCTCAAGCAAGCCGGAGACGTTGAGGAAAACCCTGGaCCcATGGCTGGATCC
GGGGACGGAGGAGCCGGCACAGCTGGGAGATGAGCGAGTTCCACAACTACAACCTGGACCTGAAGAAGAGCGACTTC
AGCACCCGGTGGCAGAAGCAGCGGTGCCCCGTGGTGAAGAGCAAGTGCCGGGAGAACGCCAGCCCCTTCTTCTTCTG
CTGCTTCATCGCCGTGGCtATGGGCATCCGGTTCATCATCATGGTGGCCATCTGGAGCGCCGTGTTCCTGAACAGCC
TGTTCAACCAGGAGGTGCAGATCCCCCTGACCGAGAGCTACTGCGGCCCCTGCCCCAAGAACTGGATCTGCTACAAG
AACAACTGCTACCAGTTCTTCGACGAGAGCAAGAACTGGTACGAGAGCCAGGCCAGCTGCATGAGCCAGAACGCCAG
CCTGCTGAAGGTGTACAGCAAGGAGGACCAGGACCTGCTGAAGCTGGTGAAGAGCTACCACTGGATGGGCCTGGTGC
ACATCCCCACCAACGGCAGCTGGCAGTGGGAGGACGGCAGCATCCTGAGCCCCAACCTGCTGACCATCATCGAGATG
CAGAAGGGCGACTGCGCCCTGTACGCCAGCAGCTTCAAGGGCTACATCGAGAACTGCAGCACCCCCAACACCTACAT
CTGCATGCAGCGGACCGTG
SEQ ID NO:76 (encoding polypeptide of SEQ ID NO: 66/Construct 8) 
ATGATCCACCTGGGCCACATCCTGTTCCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAGAGGCGGAAAAGGTCTGGGAGTGGGGCT
ACCAATTTCTCTCTCCTCAAGCAAGCCGGAGACGTTGAGGAAAACCCTGGaCCcATGGGCTGGATCCGGGGACGGAG
GAGCCGGCACAGCTGGGAGATGAGCGAGTTCCACAACTACAACCTGGACCTGAAGAAGAGCGACTTCAGCACCCGGT
GGCAGAAGCAGCGGTGCCCCGTGGTGAAGAGCAAGTGCCGGGAGAACGCCAGCCCCTTCTTCTTCTGCTGCTTCATC
GCCGTGGCtATGGGCATCCGGTTCATCATCATGGTGGCCATCTGGAGCGCCGTGTTCCTGAACAGCCTGTTCAACCA
GGAGGTGCAGATCCCCCTGACCGAGAGCTACTGCGGCCCCTGCCCCAAGAACTGGATCTGCTACAAGAACAACTGCT
ACCAGTTCTTCGACGAGAGCAAGAACTGGTACGAGAGCCAGGCCAGCTGCATGAGCCAGAACGCCAGCCTGCTGAAG
GTGTACAGCAAGGAGGACCAGGACCTGCTGAAGCTGGTGAAGAGCTACCACTGGATGGGCCTGGTGCACATCCCCAC
CAACGGCAGCTGGCAGTGGGAGGACGGCAGCATCCTGAGCCCCAACCTGCTGACCATCATCGAGATGCAGAAGGGCG
ACTGCGCCCTGTACGCCAGCAGCTTCAAGGGCTACATCGAGAACTGCAGCACCCCCAACACCTACATCTGCATGCAG
CGGACCGTGAGAAGAAAGAGAAGCGGCAGCGGCGAGGGCAGAGGCAGCCTGCTGACCTGCGGCGACGTGGAGGAGAA
CCCCGGACccATGATTCATCTCGGACATATTCTCTTTCTCTTGCTCTTGCCTGTCGCTGCCGCTCAAACAACTCCCG
GAGAAAGATCTTCTCTCCCCGCTTTTTATCCCGGAACATCTGGATCTTGTTCTGGATGTGGATCTTTGTCTCTCCCT
CTCCTCGCTGGACTCGTCGCAGCTGATGCTGTCGCTTCTCTCTTGATTGTCGGAGCTGTCTTTTTGTGTAAGAGAGG
CAGAAAGAAGCTGCTGTACATCTTCAAGCAGCCCTTCATGAGACCCGTGCAGACCACCCAGGAGGAGGACGGCTGCA
GCTGCAGATTCCCCGAGGAGGAGGAGGGCGGCTGCGAGCTG
SEQ ID NO:77 (encoding polypeptide of SEQ ID NO: 67/construct 9) 
ATGATCCACCTGGGCCACATCCTGTTCCTGCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAGAGGCGGAAAAGGTCTGGGAGTGGGGCT
ACCAATTTCTCTCTCCTCAAGCAAGCCGGAGACGTTGAGGAAAACCCTGGaCCcATGGGCTGGATCCGGGGACGGAG
GAGCCGGCACAGCTGGGAGATGAGCGAGTTCCACAACTACAACCTGGACCTGAAGAAGAGCGACTTCAGCACCCGGT
GGCAGAAGCAGCGGTGCCCCGTGGTGAAGAGCAAGTGCCGGGAGAACGCCAGCCCCTTCTTCTTCTGCTGCTTCATC
GCCGTGGCtATGGGCATCCGGTTCATCATCATGGTGGCCATCTGGAGCGCCGTGTTCCTGAACAGCCTGTTCAACCA
GGAGGTGCAGATCCCCCTGACCGAGAGCTACTGCGGCCCCTGCCCCAAGAACTGGATCTGCTACAAGAACAACTGCT
ACCAGTTCTTCGACGAGAGCAAGAACTGGTACGAGAGCCAGGCCAGCTGCATGAGCCAGAACGCCAGCCTGCTGAAG
GTGTACAGCAAGGAGGACCAGGACCTGCTGAAGCTGGTGAAGAGCTACCACTGGATGGGCCTGGTGCACATCCCCAC
CAACGGCAGCTGGCAGTGGGAGGACGGCAGCATCCTGAGCCCCAACCTGCTGACCATCATCGAGATGCAGAAGGGCG
ACTGCGCCCTGTACGCCAGCAGCTTCAAGGGCTACATCGAGAACTGCAGCACCCCCAACACCTACATCTGCATGCAG
CGGACCGTGAGAAGAAAGAGAAGCGGCAGCGGCGAGGGCAGAGGCAGCCTGCTGACCTGCGGCGACGTGGAGGAGAA
CCCCGGACcTatggctctgcctgtgacagctctgctgctgcctctggctctgctgctgcacgccgctagacccgatt
ataaggacgacgacgacaagCAAACAACTCCCGGAGAAAGATCTTCTCTCCCCGCTTTTTATCCCGGAACATCTGGA
TCTTGTTCTGGATGTGGATCTTTGTCTCTCCCTCTCCTCGCTGGACTCGTCGCAGCTGATGCTGTCGCTTCTCTCTT
 
GATTGTCGGAGCTGTCTTTTTGTGTAAGAGAGGCAGAAAGAAGCTGCTGTACATCTTCAAGCAGCCCTTCATGAGAC
CCGTGCAGACCACCCAGGAGGAGGACGGCTGCAGCTGCAGATTCCCCGAGGAGGAGGAGGGCGGCTGCGAGCTG
SEQ ID NO:78 (encoding polypeptide of SEQ ID NO: 68/construct 10) 
ATGATCCACCTGGGCCACATCCTGTTCCTGCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAGAGGCGGAAAAGGTCTGGGAGTGGGGCT
ACCAATTTCTCTCTCCTCAAGCAAGCCGGAGACGTTGAGGAAAACCCTGGaCCcATGGGCTGGATCCGGGGACGGAG
GAGCCGGCACAGCTGGGAGATGAGCGAGTTCCACAACTACAACCTGGACCTGAAGAAGAGCGACTTCAGCACCCGGT
GGCAGAAGCAGCGGTGCCCCGTGGTGAAGAGCAAGTGCCGGGAGAACGCCAGCCCCTTCTTCTTCTGCTGCTTCATC
GCCGTGGCtATGGGCATCCGGTTCATCATCATGGTGGCCATCTGGAGCGCCGTGTTCCTGAACAGCCTGTTCAACCA
GGAGGTGCAGATCCCCCTGACCGAGAGCTACTGCGGCCCCTGCCCCAAGAACTGGATCTGCTACAAGAACAACTGCT
ACCAGTTCTTCGACGAGAGCAAGAACTGGTACGAGAGCCAGGCCAGCTGCATGAGCCAGAACGCCAGCCTGCTGAAG
GTGTACAGCAAGGAGGACCAGGACCTGCTGAAGCTGGTGAAGAGCTACCACTGGATGGGCCTGGTGCACATCCCCAC
CAACGGCAGCTGGCAGTGGGAGGACGGCAGCATCCTGAGCCCCAACCTGCTGACCATCATCGAGATGCAGAAGGGCG
ACTGCGCCCTGTACGCCAGCAGCTTCAAGGGCTACATCGAGAACTGCAGCACCCCCAACACCTACATCTGCATGCAG
CGGACCGTGAGAAGAAAGAGAAGCGGCAGCGGCGAGGGCAGAGGCAGCCTGCTGACCTGCGGCGACGTGGAGGAGAA
CCCCGGACccATGATTCATCTCGGACATATTCTCTTTCTCTTGCTCTTGCCTGTCGCTGCCGCTCAAACAACTCCCG
GAGAAAGATCTTCTCTCCCCGCTTTTTATCCCGGAACATCTGGATCTTGTTCTGGATGTGGATCTTTGTCTCTCCCT
CTCCTCGCTGGACTCGTCGCAGCTGATGCTGTCGCTTCTCTCTTGATTGTCGGAGCTGTCTTTTTGTGTCAGAGGCG
GAAGTACCGGAGCAACAAGGGCGAGAGCCCCGTGGAGCCTGCCGAGCCCTGCCACTACAGCTGTCCCCGGGAGGAGG
AGGGCAGCACCATCCCCATCCAGGAGGACTACCGGAAGCCCGAGCCTGCCTGCAGCCCC
SEQ ID NO:79 (encoding polypeptide of SEQ ID NO: 69/Construct 11) 
ATGATCCACCTGGGCCACATCCTGTTCCTGCTGCTGCTGCCCGTGGCCGCTGCCCAGACCACCCCTGGCGAGCGGAG
CAGCCTGCCTGCCTTCTACCCTGGCACCAGCGGCAGCTGCAGCGGCTGCGGCAGCCTGAGCCTGCCCCTGCTGGCCG
GCCTGGTGGCCGCCGACGCCGTGGCCAGCCTGCTGATCGTGGGCGCCGTGTTCCTGTGCGCCAGGCCCAGGCGGAGC
CCtGCCCAGGAGGACGGCAAGGTGTACATCAACATGCCCGGCCGGGGCTACTTCCTGGGCAGGCTGGTGCCCAGGGG
CAGGGGCGCTGCCGAGGCTGCCACCCGGAAGCAGCGGATCACCGAGACCGAGAGCCCCTACCAGGAGCTGCAGGGCC
AGCGGAGCGACGTGTACAGCGACCTGAACACCCAGAGGCCCTACTACAAGAGGCGGAAAAGGTCTGGGAGTGGGGCT
ACCAATTTCTCTCTCCTCAAGCAAGCCGGAGACGTTGAGGAAAACCCTGGaCCcATGGGCTGGATCCGGGGACGGAG
GAGCCGGCACAGCTGGGAGATGAGCGAGTTCCACAACTACAACCTGGACCTGAAGAAGAGCGACTTCAGCACCCGGT
GGCAGAAGCAGCGGTGCCCCGTGGTGAAGAGCAAGTGCCGGGAGAACGCCAGCCCCTTCTTCTTCTGCTGCTTCATC
GCCGTGGCtATGGGCATCCGGTTCATCATCATGGTGGCCATCTGGAGCGCCGTGTTCCTGAACAGCCTGTTCAACCA
GGAGGTGCAGATCCCCCTGACCGAGAGCTACTGCGGCCCCTGCCCCAAGAACTGGATCTGCTACAAGAACAACTGCT
ACCAGTTCTTCGACGAGAGCAAGAACTGGTACGAGAGCCAGGCCAGCTGCATGAGCCAGAACGCCAGCCTGCTGAAG
GTGTACAGCAAGGAGGACCAGGACCTGCTGAAGCTGGTGAAGAGCTACCACTGGATGGGCCTGGTGCACATCCCCAC
CAACGGCAGCTGGCAGTGGGAGGACGGCAGCATCCTGAGCCCCAACCTGCTGACCATCATCGAGATGCAGAAGGGCG
 
ACTGCGCCCTGTACGCCAGCAGCTTCAAGGGCTACATCGAGAACTGCAGCACCCCCAACACCTACATCTGCATGCAG
CGGACCGTGAGAAGAAAGAGAAGCGGCAGCGGCGAGGGCAGAGGCAGCCTGCTGACCTGCGGCGACGTGGAGGAGAA
CCCCGGACcTatggctctgcctgtgacagctctgctgctgcctctggctctgctgctgcacgccgctagacccgatt
ataaggacgacgacgacaagCAAACAACTCCCGGAGAAAGATCTTCTCTCCCCGCTTTTTATCCCGGAACATCTGGA
TCTTGTTCTGGATGTGGATCTTTGTCTCTCCCTCTCCTCGCTGGACTCGTCGCAGCTGATGCTGTCGCTTCTCTCTT
GATTGTCGGAGCTGTCTTTTTGTGTCAGAGGCGGAAGTACCGGAGCAACAAGGGCGAGAGCCCCGTGGAGCCTGCCG
AGCCCTGCCACTACAGCTGTCCCCGGGAGGAGGAGGGCAGCACCATCCCCATCCAGGAGGACTACCGGAAGCCCGAG
CCTGCCTGCAGCCCC
**SEQ ID NO: 80** (A20FMDV2 peptide)
   NAVPNLRGDLQVLAQKVART
SEQ ID NO: 81 (CD124 signal peptide)
   MGWLCSGLLFPVSCLVLLQVASSGN
SEQ ID NO: 82 (CD28 aa114-220) 
IEVMYPPPYLDNEKSNGTIIHVKGKHLCPSPLFPGPSKPFWVLVVVGGVLACYSLLVTVAFIIFWVRSKRSRLLHSD
YMNMTPRRPGPTRKHYQPYAPPRDFAAYRS
SEQ ID NO: 83 (CD247 aa52-164) 
RVKFSRSADAPAYQQGQNQLYNELNLGRREEYDVLDKRRGRDPEMGGKPRRKNPQEGLYNELQKDKMAEAYSEIGMK
GERRRGKGHDGLYQGLSTATKDTYDALHMQALPPR
SEQ ID NO: 84 (SEQ ID NO: 1 of WO 2019/182425) 
MGWSCIILFLVATATGVHSQIQLVQSGPELKKPGETVKISCKTSGYTFTDYSMHWVNQAP
GKGLKWMGWINTETGEPTYTDDFKGRFAFSLETSASTAYLQINNLKNEDTATYFCARTAV
YWGQGTTLTVSSGSTSGSGKPGSGEGSDIQMTQSPSSLSASLGERVSLTCRASQEISGSL
SWLQQKPDGTIKRLIYAASTLNSGVPKRFSGRRSGSDYSLTISSLESEDFVDYYCLQYSS
YPWSFGGGTKLEIKEPKSPDKTHTCPPCPSHTQPLGVFLFPPKPKDQLMISRTPEVTCVV
VDVSQEDPEVQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAVEWES
NGQPENNYKTTPPVLDSDGSFFLYSRLTVDKSRWQEGNVFSCSVLHEALHNHYTQKSLSL
SLGKFWVLVVVGGVLACYSLLVTVAFIIFWVARPRRSPAQEDGKVYINMPGRGGRLVPRG
RGAAEAATRKQRITETESPYQELQGQRSDVYSDLNTQRPYYKRVKFSRSADAPAYQQGQN
QLYNELNLGRREEYDVLDKRRGRDPEMGGKPQRRKNPQEGLYNELQKDKMAEAYSEIGMK
GERRRGKGHDGLYQGLSTATKDTYDALHMQALPPR
SEQ ID NO: 85 (SEQ ID NO: 9 of WO 2019/182425)
   ARPRRSPAQEDGKVYINMPGRG
SEQ ID NO: 86 (SEQ ID NO: 11 of WO 2019/182425)
   GRLVPRGRGAAEAATRKQRITETESPYQELQGQRSDVYSDLNTQRPYYK

## Claims

1. A fusion polypeptide comprising:
(i) a DNAX-activating protein 10 (DAP10) polypeptide comprising the sequence of SEQ ID NO: 1, or a functional variant thereof, wherein the functional variant
(a) comprises an amino acid sequence having at least 80% sequence identity to SEQ ID NO: 1, or
(b) is a truncated version of SEQ ID NO: 1 comprising or consisting of amino acids 19-93, 19-69, 1-71, 19-71, 19-48, 49-69, 49-93, or 70-93 of SEQ ID NO: 1; and
(ii) a DNAX-activating protein 12 (DAP12) polypeptide comprising the sequence of SEQ ID NO: 9, or a functional variant thereof, wherein the functional variant
(a) comprises an amino acid sequence having at least 80% sequence identity to SEQ ID NO: 9, or
(b) is a truncated version of SEQ ID NO: 9 comprising or consisting of amino acids 22-113, 62-113, 22-61 or 41-61 of SEQ ID NO: 9.

2. The fusion polypeptide according to claim 1, having the formula, from N-terminus to C-terminus:
A-B-C-D-E,
wherein
A = an optional N-terminal sequence
B = the DAP10 polypeptide or functional variant thereof
C = an optional linker sequence
D = the DAP12 polypeptide or functional variant thereof
E = an optional C-terminal sequence.

3. The fusion polypeptide according to any previous claim, comprising the functional variant of DAP10 polypeptide, wherein the functional variant of DAP10 polypeptide comprises an amino acid sequence having at least 85%, at least 90%, at least 95%, at least 97%, at least 98% or at least 99% sequence identity to the DAP10 polypeptide of SEQ ID NO: 1.

4. The fusion polypeptide of claim 1 or claim 2, wherein the DAP10 polypeptide or functional variant thereof comprises or consists of any one of SEQ ID Nos: 1-8.

5. The fusion polypeptide according to any previous claim, comprising the functional variant of DAP12 polypeptide, wherein the functional variant of DAP12 polypeptide comprises an amino acid sequence having at least 85%, at least 90%, at least 95%, at least 97%, at least 98% or at least 99% sequence identity to the DAP12 polypeptide of SEQ ID NO: 9.

6. The fusion polypeptide of any one of claims 1-4, wherein the DAP12 polypeptide or functional variant thereof comprises or consists of any one of SEQ ID Nos: 9-13.

7. The fusion polypeptide according to any previous claim, wherein the fusion polypeptide comprises or consists of the sequence of any one of SEQ ID NOs: 60 to 63.

8. The fusion polypeptide according to claim 1 or claim 2, wherein the fusion polypeptide:
(a) does not comprise SEQ ID NO: 84; and/or
(b) does not comprise an anti-EpCAM peptide; and/or
(c) does not comprise SEQ ID NO: 85; and/or
(d) does not comprise SEQ ID NO: 86; and/or
(e) does not comprise both SEQ ID NO: 85 and SEQ ID NO: 86.

9. The fusion polypeptide according to any previous claim, wherein the fusion polypeptide is part of a contiguous chimeric polypeptide further comprising a NKG2D polypeptide or wherein the fusion polypeptide is in electrostatic association with a NKG2D polypeptide, optionally wherein the NKG2D polypeptide comprises an amino acid sequence having at least 80%, at least 85%, at least 90%, at least 95%, at least 97%, at least 98% or at least 99% sequence identity to the human NKG2D polypeptide of SEQ ID NO: 14.

10. The fusion polypeptide according to claim 9, wherein the NKG2D polypeptide is a functional variant of a NKG2D polypeptide which is a truncated version of SEQ ID NO: 14, optionally wherein the truncated version of NKG2D comprises or consists of amino acids 52-216, 73-216 or 82-216 of SEQ ID NO: 14.

11. The fusion polypeptide according to claim 9 or claim 10, wherein the fusion polypeptide is part of a contiguous chimeric polypeptide further comprising a NKG2D polypeptide and the contiguous chimeric polypeptide comprises or consists of the sequence of any one of SEQ ID NOs: 64-69.

12. An isolated nucleic acid sequence encoding the fusion polypeptide according to any previous claim, optionally wherein the isolated nucleic acid sequence comprises a nucleotide sequence having at least 80%, at least 85%, at least 90%, at least 95%, at least 97% or at least 99% sequence identity with a nucleic acid encoding any of SEQ ID NOs: 60-69 or wherein the isolated nucleic acid comprises or consists of the nucleotide sequence of any one of SEQ ID NOs: 70-79.

13. A vector comprising the nucleic acid according to claim 12, optionally wherein the vector is a lentiviral vector or a retroviral vector.

14. A host cell comprising the fusion polypeptide according to any of claims 1-11, the nucleic acid according to claim 12 or the vector according to claim 13.

15. An immunoresponsive cell selected from a T-cell or a NK cell comprising the fusion polypeptide according to any of claims 1-11, the nucleic acid according to claim 12 or the vector according to claim 13.

16. A method for making an immunoresponsive cell, comprising the steps of:
(i) transducing the nucleic acid sequence of claim 12 or vector of claim 13 into the immunoresponsive cell; and
(ii) culturing the immunoresponsive cell such that a fusion polypeptide is expressed and associates with a NKG2D polypeptide to form a CAR.

17. A pharmaceutical composition comprising the fusion polypeptide of any of claims 1-11, nucleic acid of claim 12, vector of claim 13, host cell of claim 14, or immunoresponsive cell of claim 15 and a pharmaceutically or physiologically acceptable diluent and/or carrier.

18. The fusion polypeptide of any of claims 1-11, nucleic acid of claim 12, vector of claim 13, host cell of claim 14, immunoresponsive cell of claim 15 or pharmaceutical composition according to claim 17 for use in the treatment of a pathological disorder, optionally wherein the pathological disorder is cancer.

## Patentansprüche

1. Fusionspolypeptid, umfassend:
(i) ein DNAX-aktivierendes Protein-10(DAP10-)Polypeptid, umfassend die Sequenz von SEQ ID NO: 1 oder eine funktionelle Variante davon, wobei die funktionelle Variante
(a) eine Aminosäuresequenz mit zumindest 80 % Sequenzidentität zu SEQ ID NO: 1 umfasst, oder
(b) eine verkürzte Version von SEQ ID NO: 1 umfassend oder bestehend aus Aminosäuren 19-93, 19-69, 1-71, 19-71, 19-48, 49-69, 49-93 oder 70-93 von SEQ ID NO: 1 ist; und
(ii) ein DNAX-aktivierendes Protein-12(DAP12-)Polypeptid, umfassend die Sequenz von SEQ ID NO: 9 oder eine funktionelle Variante davon, wobei die funktionelle Variante
(a) eine Aminosäuresequenz mit zumindest 80 % Sequenzidentität zu SEQ ID NO: 9 umfasst, oder
(b) eine verkürzte Version von SEQ ID NO: 9 umfassend oder bestehend aus Aminosäuren 22-113, 62-113, 22-61 oder 41-61 von SEQ ID NO: 9 ist.

2. Fusionspolypeptid nach Anspruch 1 mit der folgenden Formel von N-Terminus zu C-Terminus:
A-B-C-D-E,
wobei
A = eine optionale N-terminale Sequenz
B = das DAP10-Polypeptid oder funktionelle Variante davon
C = eine optionale Linker-Sequenz
D = das DAP12-Polypeptid oder funktionelle Variante davon
E eine optionale C-terminale Sequenz.

3. Fusionspolypeptid nach einem vorhergehenden Anspruch, umfassend die funktionelle Variante von DAP10-Polypeptid, wobei die funktionelle Variante von DAP10-Polypeptid eine Aminosäuresequenz mit zumindest 85 %, zumindest 90 %, zumindest 95 %, zumindest 97 %, zumindest 98 % oder zumindest 99 % Sequenzidentität mit dem DAP10-Polypeptid von SEQ ID NO: 1 umfasst.

4. Fusionspolypeptid nach Anspruch 1 oder Anspruch 2, wobei das DAP10-Polypeptid oder die funktionelle Variante davon ein beliebiges von SEQ ID NOs: 1-8 umfasst oder daraus besteht.

5. Fusionspolypeptid nach einem vorhergehenden Anspruch, umfassend die funktionelle Variante von DAP12-Polypeptid, wobei die funktionelle Variante von DAP12-Polypeptid eine Aminosäuresequenz mit zumindest 85 %, zumindest 90 %, zumindest 95 %, zumindest 97 %, zumindest 98 % oder zumindest 99 % Sequenzidentität mit dem DAP12-Polypeptid von SEQ ID NO: 9 umfasst.

6. Fusionspolypeptid nach einem von Anspruch 1-4, wobei das DAP12-Polypeptid oder die funktionelle Variante davon ein beliebiges von SEQ ID NOs: 9-13 umfasst oder daraus besteht.

7. Fusionspolypeptid nach einem vorhergehenden Anspruch, wobei das Fusionspolypeptid die Sequenz von einem beliebigen von SEQ ID NOs: 60 bis 63 umfasst oder daraus besteht.

8. Fusionspolypeptid nach Anspruch 1 oder Anspruch 2, wobei das Fusionspolypeptid:
(a) nicht SEQ ID NO: 84 umfasst; und/oder
(b) kein Anti-EpCAM-Peptid umfasst; und/oder
(c) nicht SEQ ID NO: 85 umfasst; und/oder
(d) nicht SEQ ID NO: 86 umfasst; und/oder
(e) weder SEQ ID NO: 85 noch SEQ ID NO: 86 umfasst.

9. Fusionspolypeptid nach einem vorhergehenden Anspruch, wobei das Fusionspolypeptid Teil eines zusammenhängenden chimären Polypeptids ist, das ferner ein NKG2D-Polypeptid umfasst, oder wobei das Fusionspolypeptid in elektrostatischer Assoziation mit einem NKG2D-Polypeptid ist, wobei optional das NKG2D-Polypeptid eine Aminosäuresequenz mit zumindest 80 %, zumindest 85 %, zumindest 90 %, zumindest 95 %, zumindest 97 %, zumindest 98 % oder zumindest 99 % Sequenzidentität mit dem menschlichen NKG2D-Polypeptid von SEQ ID NO: 14 umfasst.

10. Fusionspolypeptid nach Anspruch 9, wobei das NKG2D-Polypeptid eine funktionelle Variante eines NKG2D-Polypeptids ist, das eine verkürzte Version von SEQ ID NO: 14 ist, wobei optional die verkürzte Version von NKG2D Aminosäuren 52-216, 73-216 oder 82-216 von SEQ ID NO: 14 umfasst oder daraus besteht.

11. Fusionspolypeptid nach Anspruch 9 oder Anspruch 10, wobei das Fusionspolypeptid Teil eines zusammenhängenden chimären Polypeptids ist, das ferner ein NKG2D-Polypeptid umfasst, und das zusammenhängende chimäre Polypeptid die Sequenz von einem beliebigen von SEQ ID NOs: 64-69 umfasst oder daraus besteht.

12. Isolierte Nukleinsäuresequenz, die das Fusionspolypeptid nach einem vorhergehenden Anspruch kodiert, wobei optional die isolierte Nukleinsäuresequenz eine Nukleotidsequenz mit zumindest 80 %, zumindest 85 %, zumindest 90 %, zumindest 95 %, zumindest 97 % oder zumindest 99 % Sequenzidentität mit einer Nukleinsäure umfasst, die ein beliebiges von SEQ ID NOs: 60-69 kodiert oder wobei die isolierte Nukleinsäure die Nukleotidsequenz von einem beliebigen von SEQ ID NOs: 70-79 umfasst oder daraus besteht.

13. Vektor, umfassend die Nukleinsäure nach Anspruch 12, wobei optional der Vektor ein lentiviraler Vektor oder ein retroviraler Vektor ist.

14. Wirtszelle, umfassend das Fusionspolypeptid nach einem der Ansprüche 1-11, die Nukleinsäure nach Anspruch 12 oder den Vektor nach Anspruch 13.

15. Immunresponsive Zelle, ausgewählt aus einer T-Zelle oder einer NK-Zelle, umfassend das Fusionspolypeptid nach einem der Ansprüche 1-11, die Nukleinsäure nach Anspruch 12 oder den Vektor nach Anspruch 13.

16. Verfahren zur Herstellung einer immunresponsiven Zelle, umfassend die folgenden Schritte:
(i) Transduzieren der Nukleinsäuresequenz nach Anspruch 12 oder des Vektors nach Anspruch 13 in die immunresponsive Zelle; und
(ii) Kultivieren der immunresponsiven Zelle, sodass ein Fusionspolypeptid exprimiert wird und sich mit einem NKG2D-Polypeptid assoziiert, um einen CAR zu bilden.

17. Pharmazeutische Zusammensetzung, umfassend das Fusionspolypeptid nach einem der Ansprüche 1-11, die Nukleinsäure nach Anspruch 12, den Vektor nach Anspruch 13, die Wirtszelle nach Anspruch 14 oder die immunresponsive Zelle nach Anspruch 15 und ein pharmazeutisch oder physiologisch annehmbares Verdünnungsmittel und/oder einen pharmazeutisch oder physiologisch annehmbaren Träger.

18. Fusionspolypeptid nach einem der Ansprüche 1-11, Nukleinsäure nach Anspruch 12, Vektor nach Anspruch 13, Wirtszelle nach Anspruch 14, immunresponsive Zelle nach Anspruch 15 oder pharmazeutische Zusammensetzung nach Anspruch 17 zur Verwendung bei der Behandlung einer pathologischen Störung, wobei optional die pathologische Störung Krebs ist.

## Revendications

1. Polypeptide de fusion comprenant :
(i) un polypeptide de protéine activatrice de DNAX 10 (DAP10) comprenant la séquence de SEQ ID NO: 1, ou un variant fonctionnel de celle-ci, dans lequel le variant fonctionnel
(a) comprend une séquence d'acides aminés comportant au moins une identité de séquence de 80 % à SEQ ID NO: 1, ou
(b) est une version tronquée de SEQ ID NO: 1 constituée des acides aminés 19-93, 19-69, 1-71, 19-71, 19-48, 49-69, 49-93 ou 70-93 de SEQ ID NO: 1 ou les comprenant ; et
(ii) un polypeptide de protéine activatrice de DNAX 12 (DAP12) comprenant la séquence de SEQ ID NO: 9, ou un variant fonctionnel de celle-ci, dans lequel le variant fonctionnel
(a) comprend une séquence d'acides aminés ayant au moins 80 % d'identité de séquence à SEQ ID NO: 9, ou
(b) est une version tronquée de SEQ ID NO: 9 constituée des acides aminés 22-113, 62-113, 22-61 ou 41-61 de SEQ ID NO: 9 ou les comprenant.

2. Polypeptide de fusion selon la revendication 1, comportant la formule, de l'extrémité N-terminale à l'extrémité C-terminale :
A-B-C-D-E,
dans laquelle
A = une séquence N-terminale optionnelle
B = le polypeptide DAP10 ou un variant fonctionnel de celui-ci
C = une séquence de liaison optionnelle
D = le polypeptide DAP12 ou un variant fonctionnel de celui-ci
E = une séquence C-terminale optionnelle.

3. Polypeptide de fusion selon l'une quelconque des revendications précédentes, comprenant le variant fonctionnel du polypeptide DAP10, dans lequel le variant fonctionnel du polypeptide DAP10 comprend une séquence d'acides aminés comportant au moins 85 %, au moins 90 %, au moins 95 %, au moins 97 %, au moins 98 % ou au moins 99 % d'identité de séquence au polypeptide DAP10 de SEQ ID NO: 1.

4. Polypeptide de fusion selon la revendication 1 ou la revendication 2, dans lequel le polypeptide DAP10 ou son variant fonctionnel est constitué de l'un quelconque des SEQ ID NOs:1-8 ou le comprend.

5. Polypeptide de fusion selon l'une quelconque des revendications précédentes, comprenant le variant fonctionnel du polypeptide DAP12, dans lequel le variant fonctionnel du polypeptide DAP12 comprend une séquence d'acides aminés comportant au moins 85 %, au moins 90 %, au moins 95 %, au moins 97 %, au moins 98 % ou au moins 99 % d'identité de séquence au polypeptide DAP12 de SEQ ID NO: 9.

6. Polypeptide de fusion selon l'une quelconque des revendications 1-4, dans lequel le polypeptide DAP12 ou le variant fonctionnel de celui-ci est constitué de l'un quelconque des SEQ ID N°: 9-13 ou le comprend.

7. Polypeptide de fusion selon l'une quelconque des revendications précédentes, le polypeptide de fusion étant constitué de la séquence de l'un quelconque de SEQ ID NOs: 60 à 63 ou la comprenant.

8. Polypeptide de fusion selon la revendication 1 ou la revendication 2, le polypeptide de fusion :
(a) ne comprenant pas SEQ ID NO: 84; et/ou
(b) ne comprenant pas un peptide anti-EpCAM ; et/ou
(c) ne comprenant pas SEQ ID NO: 85 ; et/ou
(d) ne comprenant pas SEQ ID NO: 86 ; et/ou
(e) ne comprenant pas à la fois SEQ ID NO: 85 et SEQ ID NO: 86.

9. Polypeptide de fusion selon l'une quelconque des revendications précédentes, le polypeptide de fusion faisant partie d'un polypeptide chimérique contigu comprenant en outre un polypeptide NKG2D, ou le polypeptide de fusion étant en association électrostatique avec un polypeptide NKG2D, éventuellement dans lequel le polypeptide NKG2D comprend une séquence d'acides aminés comportant au moins 80 %, au moins 85 %, au moins 90 %, au moins 95 %, au moins 97 %, au moins 98 % ou au moins 99 % d'identité de séquence au polypeptide humain NKG2D de SEQ ID NO: 14.

10. Polypeptide de fusion selon la revendication 9, dans lequel le polypeptide NKG2D est un variant fonctionnel d'un polypeptide NKG2D qui est une version tronquée de SEQ ID NO: 14, éventuellement dans lequel la version tronquée de NKG2D est constituée des acides aminés 52-216, 73-216 ou 82-216 de SEQ ID NO: 14 ou les comprend.

11. Polypeptide de fusion selon la revendication 9 ou la revendication 10, le polypeptide de fusion faisant partie d'un polypeptide chimérique contigu comprenant en outre un polypeptide NKG2D et le polypeptide chimérique contigu étant constitué de la séquence de l'un quelconque de SEQ ID NOs: 64-69 ou la comprenant.

12. Séquence d'acide nucléique isolé codant le polypeptide de fusion selon l'une quelconque des revendications précédentes, la séquence d'acide nucléique isolé comprenant éventuellement une séquence de nucléotide comportant au moins 80 %, au moins 85 %, au moins 90 %, au moins 95 %, au moins 97 % ou au moins 99 % d'identité de séquence avec un acide nucléique codant l'un quelconque des SEQ ID NOs: 60-69 ou l'acide nucléique isolé étant constitué de la séquence nucléotide de l'un quelconque de SEQ ID NOs: 70-79 ou la comprenant.

13. Vecteur comprenant l'acide nucléique selon la revendication 12, le vecteur étant éventuellement un vecteur lentiviral ou un vecteur rétroviral.

14. Cellule hôte comprenant le polypeptide de fusion selon l'une quelconque des revendications 1-11, l'acide nucléique selon la revendication 12, ou le vecteur selon la revendication 13.

15. Cellule immunoréactive sélectionnée entre un lymphocyte T et un lymphocyte NK comprenant le polypeptide de fusion selon l'une quelconque des revendications 1-11, l'acide nucléique selon la revendication 12, ou le vecteur selon la revendication 13.

16. Procédé de fabrication d'une cellule immunoréactive, comprenant les étapes de :
(i) transduction de la séquence d'acide nucléique selon la revendication 12 ou du vecteur selon la revendication 13 dans la cellule immunoréactive ; et
(ii) mise en culture de la cellule immunoréactive de telle sorte qu'un polypeptide de fusion soit exprimé et s'associe avec un polypeptide NKG2D pour former un CAR.

17. Composition pharmaceutique comprenant le polypeptide de fusion selon l'une quelconque des revendications 1-11, l'acide nucléique selon la revendication 12, le vecteur selon la revendication 13, la cellule hôte selon la revendication 14, ou la cellule immunoréactive selon la revendication 15 et un diluant et/ou excipient pharmaceutiqument acceptable ou physiologiquement acceptable.

18. Polypeptide de fusion selon l'une quelconque des revendications 1-11, acide nucléique selon la revendication 12, vecteur selon la revendication 13, cellule hôte selon la revendication 14, cellule immunoréactive selon la revendication 15 ou composition pharmaceutique selon la revendication 17 destiné(e) à être utilisé(e) dans le traitement d'un trouble pathologique, le trouble pathologique étant éventuellement un cancer.
